# EUROPEAN PATENT APPLICATION

(11) **EP 4 806 342 A1**
(43) Date of publication of application: **16.09.2026**
(21) Application number: 25208933.9
(22) Date of filing: 15.10.2025
(51) Int. Cl.: A61B 5/0205, A61B 5/024, A61B 5/11, A61B 5/389, A61B 5/00

(54) **A WEARABLE DEVICE FOR DETECTING A USER STATE OF A USER AND A METHOD THEREOF**

(30) Priority: 11.03.2025 IN 202541021987
(71) Applicant: Ultrahuman Healthcare Private Limited, Bangalore South, Karnataka 560068 (IN)
(72) Inventor: SHARMA, Anant, 110075 New Delhi (IN); SHREY, Shwetank, 800013 Bihar (IN); KUSHWAHA, Ayush, 110019 New Delhi (IN); PURANAM, Akash Raja, 560109 Karnataka (IN); KARKERA, Satvik, 575001 Karnataka (IN); KUMAR, Mohit, Abu Dhabi (AE); SINGHAL, Vatsal, Abu Dhabi (AE)
(74) Representative: Office Freylinger

(57) **Abstract**

The present disclosure relates to a wearable device [100] for detecting a user state of a user and a method thereof. The wearable device [100] comprises a data processing module [102] and a data collection module [108], for detecting in real-time fatigue measurement and muscle activity even with minimal limb movement. The present disclosure provides obtaining a set of target data associated with the user by the data collection module [108] and thereafter extracting from it a set of target features by the processing unit [106]. Thereafter, based on the set of target features, a fused set of features are generated and then classified into one or more categories. The one or more categories comprises a fatigue level category, a user activity category and/or a user health category based on which the user state is determined.

## Description

### TECHNICAL FIELD

The present disclosure relates to the field of health and fitness monitoring technologies. Particularly, the present disclosure relates to the field of sensor-based devices for health and fitness monitoring. More particularly, the present disclosure relates to a wearable device for detecting a user state of a user and a method thereof.

### BACKGROUND

The following description of the related art is intended to provide background information pertaining to the field of the disclosure. This section may include certain aspects of the art that may be related to various features of the present disclosure. However, it should be appreciated that this section is used only to enhance the understanding of the reader with respect to the present disclosure, and not as admissions of the prior art.

There is an increasing trend in usage of a wearable apparatus by a user for tracking health and fitness. The wearable apparatus is an electronic apparatus that is designed to be used for tracking health and fitness of the user while being worn by said user. In today's digital age, one or more types of wearable apparatus are available such as smart rings, smartwatches, fitness trackers, wrist bands, etc. Such wearable apparatus may comprise one or more wearable devices such as a fastener used for fastening the wearable apparatuses, some examples of the wearable device may be a band or strap for fastening the wearable apparatus to the part of body of the wearable apparatus. Further, the wearable apparatus is equipped with a bunch of sensors for tracking health and fitness activities of the users, such as, an accelerometer, a gyroscope, inertial measurement units (IMUs), a Photoplethysmography (PPG) sensors, etc. These sensors are responsible for measuring a set of data related to the health of the user of a wearable device such as heart rate, oxygen saturation, respiration rate, blood pressure, etc. of the user of the wearable apparatus.

Conventionally, for detecting various activities of the users, the wearable apparatus primarily relies on the IMUs and the PPG sensors. In conventional wearable apparatus, the time required for recovery and the efforts exerted by a user during exercise are measured based on the user's heart rate data collected by the PPG sensors embedded in such wearable apparatus. Further, the IMUs embedded in such conventional wearable apparatus, play a crucial role in detecting activities and counting repetitions, which are essential for identifying fatigue and tracking user motion. However, the existing solutions based on IMUs struggle to detect muscle activation levels and differentiate between complex activities performed by the user such as strength training exercises.

It is to be noted that a muscle operation causes changes in motion, but metrics like fatigue are difficult to track directly through their cause i.e., cause of change in the motion due to a particular muscle group being operated by the user. Instead, they are monitored through their effects such as change in the heart rate due to the motion when a muscle group being operated by the user. The relationship between the cause and effect of fatigue varies among individuals, complicating accurate tracking. While heart rate is used as a primary physiological metric to monitor recovery, continuous tracking of muscle recovery remains elusive in currently known consumer wearable apparatus. Thus, there exists a need for a solution and/or a wearable apparatus that correctly tracks the recovery of the muscles.

Further, in the existing wearable apparatus rely solely on the data collected from the accelerometer and the gyroscope, making said wearable apparatus inaccurate for differentiating similar exercises or detecting isometric contractions. Therefore, the existing solutions cannot distinguish between similar-looking movements (e.g., a bicep curl vs. a hammer curl) and fail to capture isometric exercises with minimal limb displacement.

Furthermore, the existing solutions also fails to integrate the IMU data and the EMG data in real-time and therefore are unable to classify the exercises with minimal limb displacement that are being performed by the user, and/or detect the activity of the user accurately and/or monitor the fatigue of the user.

Therefore, there are a number of limitations to the existing solutions and in order to overcome these and such other limitations of the known solutions it is necessary to provide a device for real-time monitoring of health and fitness of the user by integrating data from multiple sensors such as the IMU data, the EMG data and the PPG data in real-time.

### SUMMARY

This section is provided to introduce certain aspects of the present disclosure in a simplified form that are further described below in the detailed description. This summary is not intended to identify the key features or the scope of the claimed subject matter.

An aspect of the present disclosure relates to a wearable device for detecting a user state of a user. The wearable device comprises a data collection module and a data processing module. The data collection module is configured to collect data from a set of sensors. The data processing module is connected at least to the data collection module. The data processing module comprises a memory unit and a processing unit. The processing unit is configured to obtain, from a data collection module, a set of target data associated with the user. The target data comprises at least two from among a set of movement data, a set of muscle-activity data, a set of heart-rate data and a set of temperature data. The processing unit is further configured to extract a set of target features from the set of target data, wherein the set of target features comprises at least two from among a set of movement features, a set of muscle-activity features, a set of heart-rate features and a set of temperature features. The processing unit is further configured to generate a fused set of features based on a combination of two or more target features from the set of target features. The processing unit is further configured to classify the fused set of features into one or more categories. The one or more categories comprises at least one of a fatigue level category, a user activity category and a user health category. Further, the processing unit is configured to determine the user state based on the one or more categories.

In an exemplary aspect of the present disclosure, the user state indicates at least one of a fatigue level for the user, a movement of the user, an activity of one or more muscles of the user and a health state of the user.

In another exemplary aspect of the present disclosure, the fatigue level of the user is determined based on the fatigue level category, the movement of the user is determined based the user activity category, the activity of one or more muscles of the user is determined based on the user activity category, and the health state of the user is determined based on the user health category.

In another exemplary aspect of the present disclosure, the set of movement features is extracted from the set of movement data, the set of muscle-activity features is extracted from the set of muscle-activity data, the set of heart-rate features is extracted from the set of heart-rate data, and the set of temperature features is extracted from the set of temperature data.

In another exemplary aspect of the present disclosure, the fused set of features is generated by the processing unit based on a target technique using a first artificial- intelligence (AI)-based subsystem.

In another exemplary aspect of the present disclosure, the first AI-based subsystem is trained based on at least one of a historical fused set of features generated from a historical set of target features, and wherein the historical set of target features comprises at least two from among a historical set of movement features, a historical set of muscle-activity features, a historical set of heart-rate features and a historical set of temperature features.

In another exemplary aspect of the present disclosure, the one or more categories are generated by the processing unit using a second artificial- intelligence (AI) based subsystem.

In another exemplary aspect of the present disclosure, the second AI-based subsystem is trained based on one or more historical categories. The one or more historical categories are classified based on a historical fused set of features, Further, the one or more historical categories comprises at least one of a historical fatigue level category, a historical user activity category and a historical user health category.

In another exemplary aspect of the present disclosure, the data collection module comprises the set of sensors selected from among an inertial measurement unit (IMU), an electromyography (EMG) sensor, a photoplethysmography (PPG) sensor, and a temperature sensor.

In another exemplary aspect of the present disclosure, prior to extracting the set of target features from the set of target data, the processing unit is configured to perform at least one of a data pre-processing technique and a data segmentation technique on the set of target data.

Another aspect of the present disclosure may relate to a method for detecting a user state of a user. The method comprises obtaining, by a processing unit from a data collection module, a set of target data associated with the user. The set of target data comprises at least two from among a set of movement data, a set of muscle-activity data, a set of heart-rate data and a set of temperature data. The method further comprises extracting, by the processing unit, a set of target features from the set of target data, wherein the set of target features comprises at least two from among a set of movement features, a set of muscle-activity features, a set of heart-rate features and a set of temperature features. The method further comprises generating, by the processing unit, a fused set of features based on a combination of two or more target features from the set of target features. The method further comprises classifying, by the processing unit, the fused set of features into one or more categories, wherein the one or more categories comprises at least one of a fatigue level category, a user activity category and a user health category. Further, the method comprises determining, by the processing unit, the user state based on the one or more categories.

In another exemplary aspect of the present disclosure, the user state indicates at least one of a fatigue level for the user, a movement of the user, an activity of one or more muscles of the user and a health state of the user.

In another exemplary aspect of the present disclosure, the fatigue level of the user is determined based on the fatigue level category, the movement of the user is determined based the user activity category, the activity of one or more muscles of the user is determined based on the user activity category, and the health state of the user is determined based on the user health category.

In another exemplary aspect of the present disclosure, the set of movement features is extracted from the set of movement data, the set of muscle-activity features is extracted from the set of muscle-activity data, the set of heart-rate features is extracted from the set of heart-rate data, and the set of temperature features is extracted from the set of temperature data.

In another exemplary aspect of the present disclosure, the fused set of features is generated by the processing unit based on a target technique using a first artificial- intelligence (AI)-based subsystem.

In another exemplary aspect of the present disclosure, the first AI-based subsystem is trained based on at least one of a historical fused set of features generated from a historical set of target features, and wherein the historical set of target features comprises at least two from among a historical set of movement features, a historical set of muscle-activity features, a historical set of heart-rate features and a historical set of temperature features.

In another exemplary aspect of the present disclosure, the one or more categories are generated by the processing unit using a second artificial-intelligence (AI) based subsystem.

In another exemplary aspect of the present disclosure, the second AI-based subsystem is trained based on one or more historical categories. The one or more historical categories are classified based on a historical fused set of features. Further, the one or more historical categories comprises at least one of a historical fatigue level category, a historical user activity category and a historical user health category.

In another exemplary aspect of the present disclosure, the data collection module comprises one or more sensors selected from among an inertial measurement unit (IMU), an electromyography (EMG) sensor, a photoplethysmography (PPG) sensor, and a temperature sensor.

In another exemplary aspect of the present disclosure, prior to extracting the set of target features from the set of target data, the processing unit performs on the set of target data at least one of a data pre-processing technique and a data segmentation technique.

Yet another aspect of the present disclosure may relate to a non-transitory computer readable storage medium storing one or more instructions for detecting a user state of a user, the one or more instructions include executable code which, when executed by one or more units of a wearable device, causes a processing unit of the wearable device to obtain, from a data collection module, a set of target data associated with the user. The target data comprises at least two from among a set of movement data, a set of muscle-activity data, a set of heart-rate data and a set of temperature data. Further, the one or more instructions when executed causes the processing unit to extract a set of target features from the set of target data, wherein the set of target features comprises at least two from among a set of movement features, a set of muscle-activity features, a set of heart-rate features and a set of temperature features. Further, the one or more instructions when executed causes the processing unit to generate a fused set of features based on a combination of two or more target features from the set of target features. Further, the one or more instructions when executed causes the processing unit to classify the fused set of features into one or more categories. The one or more categories comprises at least one of a fatigue level category, a user activity category and a user health category. Further, the one or more instructions when executed causes the processing unit to determine the user state based on the one or more categories.

### OBJECTS OF DISCLOSURE

Some of the objects of the present disclosure which at least one embodiment disclosed herein satisfies are listed below.

It is an object of the present disclosure to provide a wearable device for monitoring health and fitness of a user in real-time and a method thereof.

It is another object of the present disclosure to provide a wearable device for detecting a muscle activity having minimal limb displacement.

It is another object of the present disclosure to provide a solution to integrate in real-time data from multiple sensors of the wearable device for monitoring health and fitness of a user in real-time.

It is yet another object of the present disclosure to provide a solution to improve activity detection, activity monitoring, activity classification, recovery monitoring, and health monitoring.

It is yet another object of the present disclosure to provide a solution that combines EMG and IMU signals in real-time to accurately detecting a user activity with minimal limb displacement.

It is yet another object of the present disclosure to provide a solution that integrates data from multiple sensors to accurately monitor physical activity, recovery period associated with a user based on the performed physical activity performed by the user and a sleep parameter associated with the user.

It may be noted that the above-mentioned objectives are only exemplary for understanding the context of the present disclosure and shall not be construed to be limiting in nature. Any other objectives appreciated by a person skilled in the art in light of the present disclosure shall also be construed to be well within the scope of the present disclosure.

### BRIEF DESCRIPTION OF DRAWINGS

The accompanying drawings, which are incorporated herein, constitute a part of this disclosure. Components in the drawings are not necessarily as per scale, and the emphasis instead has been placed upon clearly illustrating the principles of the present disclosure. Some drawings may indicate the components using block diagrams and may not represent the internal circuitry of each component. It will be appreciated by those skilled in the art that disclosure of such drawings includes disclosure of electrical components or circuitry commonly used to implement such components. Although exemplary connections between sub-components have been shown in the accompanying drawings, it will be appreciated by those skilled in the art that other connections may also be possible, without departing from the scope of the disclosure. All sub-components within a component may be connected to each other, unless otherwise indicated.
FIG. 1 illustrates an exemplary block diagram of a wearable device for detecting a user state of a user, in accordance with the exemplary embodiments of the present disclosure.
FIG. 2 illustrates a perspective view of a wearable apparatus in accordance with exemplary embodiments of the present disclosure.
FIG. 3A illustrates a side view of the wearable apparatus in accordance with exemplary embodiments of the present disclosure.
FIG. 3B illustrates another side view of the wearable apparatus in accordance with exemplary embodiments of the present disclosure.
FIG. 4 illustrates an exemplary flow diagram of a method for detecting the user state of the user, in accordance with the exemplary implementations of the present disclosure.

The foregoing shall be more apparent from the following more detailed description of the disclosure.

### DETAILED DESCRIPTION

In the following description, for the purposes of explanation, various specific details are set forth in order to provide a thorough understanding of embodiments of the present disclosure. It will be apparent, however, that embodiments of the present disclosure may be practiced without these specific details. Several features described hereafter may each be used independently of one another or with any combination of other features. An individual feature may not address any of the problems discussed above or might address only some of the problems discussed above.

The ensuing description provides exemplary embodiments only, and is not intended to limit the scope, applicability, or configuration of the disclosure. Rather, the ensuing description of the exemplary embodiments will provide those skilled in the art with an enabling description for implementing an exemplary embodiment. It should be understood that various changes may be made in the function and arrangement of elements without departing from the spirit and scope of the disclosure as set forth.

Specific details are given in the following description to provide a thorough understanding of the embodiments. However, it will be understood by one of ordinary skills in the art that the embodiments may be practiced without these specific details. For example, circuits, systems, processes, and other components may be shown as components in block diagram form in order not to obscure the embodiments in unnecessary detail.

Also, it is noted that individual embodiments may be described as a process which is depicted as a flowchart, a flow diagram, a data flow diagram, a structure diagram, or a block diagram. Although a flowchart may describe the operations as a sequential process, many of the operations may be performed in parallel or concurrently. In addition, the order of the operations may be re-arranged. A process is terminated when its operations are completed but could have additional steps not included in a figure.

The word "exemplary" and/or "demonstrative" is used herein to mean serving as an example, instance, or illustration. For the avoidance of doubt, the subject matter disclosed herein is not limited by such examples. In addition, any aspect or design described herein as "exemplary" and/or "demonstrative" is not necessarily to be construed as preferred or advantageous over other aspects or designs, nor is it meant to preclude equivalent exemplary structures and techniques known to those of ordinary skill in the art. Furthermore, to the extent that the terms "includes," "has," "contains," and other similar words are used in either the detailed description or the claims, such terms are intended to be inclusive-in a manner similar to the term "comprising" as an open transition word-without precluding any additional or other elements.

As used herein, an "apparatus" or an "electronic apparatus" or a "wearable apparatus" may refer to a set of devices used for the implementation of the technical solution provided by the present disclosure. Said apparatus may comprise one or more wearable devices that may either individually or collectively perform one or more functions for implementing the technical solutions of the present disclosure. The term "wearable apparatus" may refer to an apparatus that may be worn by a user of the wearable apparatus. Further, as used herein, the term "device" or "wearable device" or "electronic device" may further comprise one or more components or modules, which may be further used for implementing the technical solution of the present disclosure. For example, the wearable apparatus may be a smartwatch, and the wearable device may refer to a strap used for fastening the smartwatch on the hand of the user.

As used herein an "electromyography sensor" or "EMG sensor" may refer to a component or a sensor module used for sensing one or more signals relating to movement of one or more muscles of the user. The EMG sensor may include, but not limited to, a single channel EMG sensor or a multi-channel EMG sensor.

As used herein an "inertial measurement unit" or "IMU" or "IMU sensor" may refer to a component or a sensor module used for sensing one or more signals relating to movement of the one or more users. The IMU sensor may comprise, but not limited to, one or more of accelerometers and/or one or more of gyroscopes which may be used for detection of accelerometer data and gyroscope data for determining the velocity and orientation of the user.

As used herein, a "photoplethysmography sensor" or "PPG sensor" or "PPG device" may refer to a component or a sensor module used for sensing one or more signals relating to the variations in the blood flow. The PPG sensor senses the variations in the intensity of light that is transmitted through or reflected from the tissue. These intensity changes are associated with changes in blood flow through the tissue and provide vital cardiovascular information such as the pulse rate. The PPG sensor may comprise a high-intensity green light source such as from a green light emitting diode. The PPG sensor may also comprise other light source such as a red light source or an infrared light source comprising visible light, infrared or ultraviolet radiation, X-rays and gamma rays configured to sense signals relating to the variations in the blood flow.

As used herein, a "module" or a "processing unit" includes one or more processors, wherein processor refers to any logic circuitry for processing instructions. A processor may be a general-purpose processor, a special purpose processor, a conventional processor, a digital signal processor, a plurality of microprocessors, one or more microprocessors in association with a DSP core, a controller, a microcontroller, Application Specific Integrated Circuits, Field Programmable Gate Array circuits, any other type of integrated circuits, etc. The processor may perform signal coding, data processing, input/output processing, and/or any other functionality that enables the working of the system according to the present disclosure. More specifically, the processor or processing unit is a hardware processor.

As used herein, "a user device" may be any electrical, electronic and/or computing device or equipment, capable of implementing the features of the present disclosure. The user equipment/device may include, but is not limited to, a mobile phone, smart phone, laptop, a general-purpose computer, desktop, personal digital assistant, tablet computer, wearable device or any other computing device which is capable of implementing the features of the present disclosure. Also, the user device may contain at least one input means configured to receive an input from at least one of a transceiver unit, a processing unit, a storage unit, a detection unit and any other such unit(s) which are required to implement the features of the present disclosure.

As used herein, the "user device" and/or "module" may comprise at least "storage unit" or "memory unit", wherein "storage unit" or "memory unit" refers to a machine or computer-readable medium including any mechanism for storing information in a form readable by a computer or similar machine. For example, a computer-readable medium includes read-only memory ("ROM"), random access memory ("RAM"), magnetic disk storage media, optical storage media, flash memory devices or other types of machine-accessible storage media. The storage unit stores at least the data that may be required by one or more units of the system to perform their respective functions.

As used herein the expression "at least one of" shall be interpreted as an inclusive term that includes at least one of the succeeding elements, and shall also include multiple of such elements in different combinations. For example, the term "an exemplary parameter comprising at least one of A, B and C" may imply that the exemplary parameter may comprise only {A} or only {B} or only {C}, or {A, B, C} collectively, and may also comprise various combinations of A, B, and C such as {A, B}, {B, C} or {C, A}. Further, the expression shall also be construed to be include multiple instances of such elements for example {A, A}, {A, B, C, A, B, C}, etc. It should be noted that the terms "first", "second", "primary", "secondary", "target" and the like, herein do not denote any order, ranking, quantity, or importance, but rather are used to distinguish one element from another.

As used herein the expression "a set of' shall be interpreted as a collection of dataset or elements. The set may include a finite set with one element or more than one element. In an example, a set of elements may be made by an array of elements. For example, "a set of X" includes, {X}, {XX} and/or {X1, X2, X3}. Further, "the set of X" may also include an element Y that may or may not be related to X unless repugnant to the context thereof.

As used herein, the expression "and/or" includes any single item from items or a combination of items associated with the items. For example, a group of A, B and/or C includes only A, only B, only C, a combination of A and B, a combination of B and C, a combination of A and C or a combination of A, B and C.

As used herein, the expression "one or more" or "one or more of" includes any single item in the list or a combination of items in the list. For example, one or more of A, B and C includes only A, only B, only C, a combination of A and B, a combination of B and C, a combination of A and C, a combination of A, B and C, a combination of multiple A and multiple B, a combination of multiple A, a single B and a single C, a combination of single A, multiple B and multiple C and any other such like combinations.

As discussed in the background section, the current known solutions have shortcomings such as they perform incorrect activity exercise classification, activity detection, activity monitoring, recovery monitoring, health monitoring and fatigue monitoring in real-time. Further, the known solutions are unable to integrate data from multiple sensors in real-time, for example, the conventional approaches fail to integrate data collected by an inertial measurement unit (IMU), an electromyography (EMG) sensor and/or photoplethysmography (PPG) sensor in real-time. Further, the conventional approaches are unable to detect and classify the activity performed by the user that may be used for real-time monitoring of the health and fitness of the user. The present disclosure aims to overcome the above-mentioned and other existing problems in this field of technology by providing a wearable device for detecting a user state of a user and a method thereof which facilitates monitoring of the health and fitness of the user in real-time. The present disclosure provides a novel and inventive solution for detecting and classifying in real-time, the activities performed by the user and to accurately detect and distinguish in real-time, the activities involving minimal limb movement while monitoring fatigue, muscle recovery, etc of the user.

Hereinafter, exemplary embodiments of the present disclosure will be described with reference to the accompanying drawings.

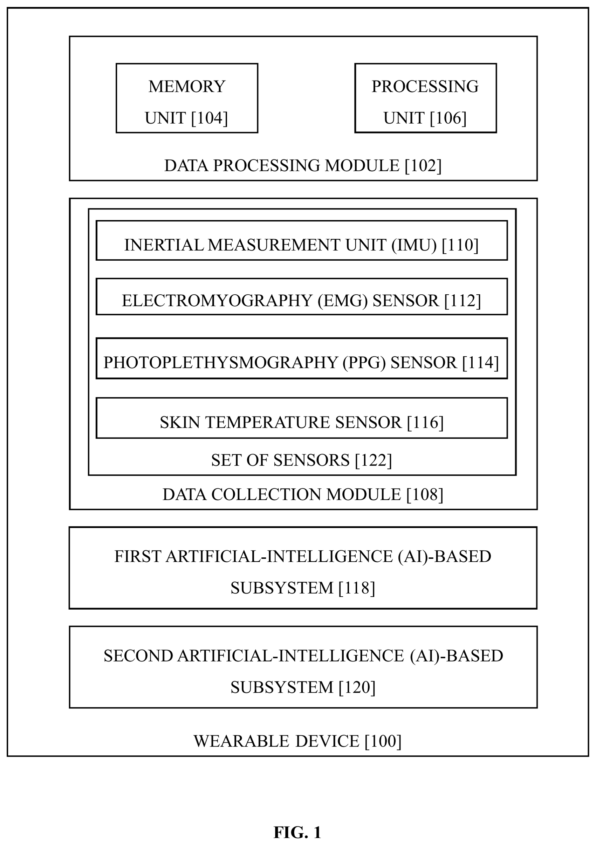

**FIG. 1** illustrates an exemplary block diagram representation of a wearable device [100] for detecting a user state of a user, in accordance with exemplary embodiments of the present disclosure. The wearable device [100] comprises at least a data processing module [102] and a data collection module [108] to implement the present disclosure.

As shown in the figure, the data processing module [102] may comprise at least a memory unit [104] and a processing unit [106]. In an exemplary embodiment of the present disclosure as disclosed herein, the memory unit [104] may be a flash memory which is used for local storage. In another exemplary embodiment of the present disclosure as disclosed herein, the processing unit [106] may be a microcontroller which may be used for signal acquisition, feature extraction and machine learning based classification. In a non-limiting preferred embodiment of the present disclosure as disclosed herein, the memory unit [104] and the processing unit [106] are communicatively coupled to each other in a manner as obvious to a person skilled in the art for implementing features of the present disclosure. Also, in FIG. 1 only a few units are shown, however, the data processing module [102] and the data collection module [108] may comprise multiple such units, as may be required to implement the features of the present disclosure. Further, in an exemplary embodiment, the data processing module [102] may reside in and/or connected to and/or in communication with a user device (may also be referred herein as a user equipment or a UE) to implement the features of the present disclosure.

The data collection module [108] of the wearable device [100] may refer to a module configured to collect data from a set of sensors [122]. In one implementation, the data collection module [108] may comprise the set of sensors [122]. The set of sensors [122] may be such as one or more of an inertial measurement unit (IMU) [110], an electromyography (EMG) sensor [112], a Photoplethysmography (PPG) sensor [114], a temperature sensor [116], etc. In another implementation, the data collection module [108] may be communicatively connected to such set of sensors [122] for collection of the data such as in an event at least one of the set of sensors [122] are not present in the data collection module.

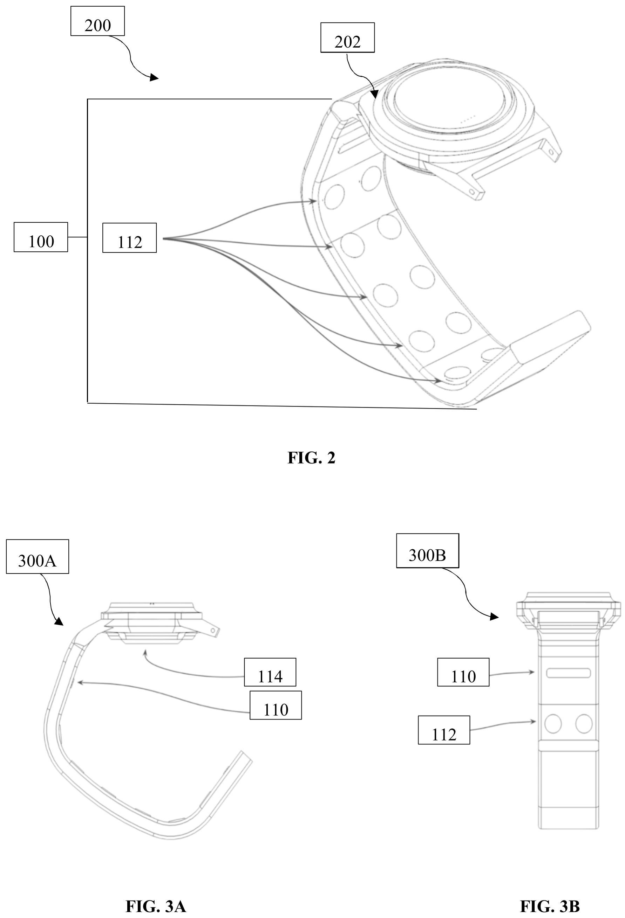

Referring to **FIG. 2****,** wherein the FIG.2 illustrates a perspective view of a wearable apparatus [200], in accordance with exemplary implementations of the present disclosure. Referring to **FIG. 3A,** and **FIG. 3B****,** wherein side views of the wearable apparatus [200] is shown in accordance with exemplary implementations of the present disclosure. For ease of understanding, the FIG. 2, FIG. 3A and FIG. 3B are explained in conjunction in the foregoing description for explanation of the technical solution as disclosed by the present disclosure. As shown in the FIG. 2, FIG. 3A and FIG. 3B, the wearable apparatus [200] comprises at least a first component [202], and a wearable device [100] connected to the first component [202] of the wearable apparatus [200]. The first component [202] may refer to a housing for storing one or more electronic components for enabling various functionalities of the wearable apparatus [200]. The wearable device [100] may comprise a material that may be used for wrapping the wearable apparatus [200] around the body of the user, for example, a band used for fastening the wearable apparatus [200] on the wrist of the user. As would be understood to a person skilled in the art, the first component [202] may be provided with a groove and/or a fastening mechanism for coupling the wearable device [100] with the first component [202].

In an exemplary non-preferred implementation, the wearable apparatus [200] may comprise one or more modules or components to implement the solution of the present disclosure. In one implementation, a data collection module [108] of the wearable device [100] may comprise at least two from among: an inertial measurement unit (IMU) [110], an electromyography (EMG) sensor [112], a Photoplethysmography (PPG) sensor [114], a temperature sensor [116], etc.

The inertial measurement unit (IMU) [110] may be a component used for collecting information associated with a set of movement data associated with the user. The IMU [110] may sense a multi-axis acceleration from an accelerometer and a gyroscope data from a gyroscope that can be combined to form the set of movement data. In one embodiment of the present disclosure, the IMU [110] may be provided on a smart clothing apparel. In one exemplary embodiment of the present disclosure, the IMU [110] may comprise a 3-axis accelerometer that may collect the set of movement data at a frequency of 100-200 Hz. In another exemplary embodiment of the present disclosure, the IMU [110] may comprise a 3-axis gyroscope that may collect the set of movement data at a frequency of 100-200 Hz.

The EMG sensor [112] may refer to a sensor module used for tracking a set of muscle-activity data associated with the user. The EMG sensor [112] may implement electromyography (EMG) technique in which the EMG sensor [112] measures muscle response or electrical activity in response to a nerve's stimulation of the muscle. In one embodiment, there may be a plurality of EMG sensor [112] configured to the wearable device [100], wherein each of the EMG sensor [112] would enable precise analysis of the activity of the one or more muscles of the user. In some embodiments, the EMG sensor [112] of the wearable device [100] may also be provided on the smart clothing apparel. In an exemplary embodiment, the EMG sensor [112] may comprise surface electrodes that may collect the set of muscle-activity data at a frequency of 20-250 Hz.

The PPG sensor [114] may refer to a sensor used for sensing a heart-rate data associated with the user. The PPG sensor [114] may sense a heart rate and an oxygen level in blood. The temperature sensor [116] may refer to a sensor used to sense a set of temperature data associated with a temperature of a skin of the user that is proximal to the wearable device [100]. The temperature sensor [116] may be configured to monitor physiological stress levels based on the temperature of the skin. In an exemplary embodiment of the present disclosure, the PPG sensor [114] may comprise a multi-wavelength light-emitting diode (LED) and/or a photodiode that may collect the set of heart-rate data at a frequency of 25 - 100 Hz. Further, in another exemplary embodiment of the present disclosure, the temperature sensor [116] may comprise a thermistor or an infrared (IR) sensor that may collect the set of heart-rate data at a frequency of 0.5 - 2 Hz.

In a non-limiting implementation of the present disclosure, the PPG sensor [114] and the temperature sensor [116] may be integrated with the wearable device [100]. Further, in another non-limiting implementation of the present disclosure, the PPG sensor [114] and the temperature sensor [116] may be provided on the first component [202] of the wearable apparatus [200] as has also been shown in FIG. 3A and 3B. In such non-limiting implementation(s), the data collection module [108] of the wearable device [100] may be connected with the PPG sensor [114] and the temperature sensor [116] of the first component [202] to collect the set of heart-rate data and the set of temperature data. In another implementation, the wearable device [100] may also comprise power management integrated circuits (PMIC) and a charging apparatus for power management. The composition of the wearable device [100] as disclosed above shall not be construed in any manner to limit the scope of the present disclosure and the wearable device [100] may also comprise any additional components as may be understood to a person skilled in the art while referring the present disclosure, that may be required for enabling the functions of the present invention.

In one embodiment of the present disclosure, the PPG sensor [114] may be embedded in the wearable device [100]. In said embodiment, the wearable device [100] may collect the set of heart-rate data such as data related to the heart rate, heart rate variability (HRV), oxygen saturation (SpO2) and peripheral circulation of the user, which is then used to track recovery and exercise efforts.

In another embodiment of the present disclosure, the PPG sensor [114] may be embedded in portion of the wearable apparatus [200] other than the wearable device [100]. In said embodiment, the wearable apparatus [200] may collect the set of heart-rate data such as data related to the heart rate, the HRV, the SpO2 and the peripheral circulation of the user. In such embodiments, the wearable device [100] may retrieve the data from the wearable apparatus [200] related to the heart rate, the HRV, the SpO2 and the peripheral circulation of blood of the user, thereafter the wearable device [100] may utilise such retrieved data to track recovery and exercise efforts. This setup allows for flexibility in the type of wearable technology used to implement the solution of the present disclosure.

Further, in both embodiments as discussed above, the PPG sensor [114] may utilize the set of heart-rate data related to the heart rate, the HRV, the SpO2 and the peripheral circulation of the user to monitor changes in blood volume, providing a continuous and unobtrusive measurement of heart rate so as to assess cardiovascular health of the user and optimize activity/workout routines of the user.

The wearable device [100] is configured for detecting a user state of a user, with the help of the interconnection between the components/units of the system [300]. The user may wear the wearable device [100] on any part of their body for detecting the user state through the wearable device [100]. The user state may refer to a physiological state of the user that indicates the health and fitness of the user. The physiological state may refer to the condition of the body's physical functions encompassing various processes incurred by user's body and their impact on overall well-being of the user, for example working out for longer period of time may result in sustained exertion by the user indicating fatigue as the user state, similarly, higher resting heart-rate may indicate stress as the user state.

In an implementation of the present disclosure, the user state may indicate at least one of a fatigue level for the user, a movement of the user, an activity of one or more muscles of the user and a health state of the user. The fatigue level for the user may refer to a level of tiredness experienced by the user or in any body part of the user, for example, high tiredness, muscle twitching, etc. The movement of the user indicates the activity performed by the user, for example, a workout movement, a running movement, etc. The activity of the one or more muscles of the user may refer to an activity performed by a particular group of muscles forming a part of the body, for example, a bicep workout, a legs workout, etc. The health state of the user may refer to a condition of the body part of the user that indicates the healthiness of a particular body part, for example, frequent muscle twitching may indicate bad health state of the user.

In operation, for detecting the user state of the user, the processing unit [106] is configured to obtain from the data collection module [108], a set of target data associated with the user. The set of target data comprises at least two from among a set of movement data, a set of muscle-activity data, a set of heart-rate data and a set of temperature data. As disclosed above, the data collection module [108] collects data from the set of sensors [122] and then transmits the sensed data to the processing unit [106]. In one exemplary implementation of the present disclosure, the data collection module [108] may comprise a shared clock or a synchroniser that helps match the time at which the data is sensed by the one or more sensors. In another exemplary implementation of the present disclosure, the set of movement data, the set of muscle-activity data, the set of heart-rate data and the set of temperature data are raw signals received from the set of sensors [122]. For example, the set of movement data may be collected from the IMU [110], the set of muscle-activity data may be collected from the EMG sensor [112], the set of heart-rate data may be collected from the PPG sensor [114], the set of temperature data may be collected from the temperature sensor [116]. The raw signal may refer to an electrical signal associated with the user that may be sensed by the set of sensors [122] during the detection of the user state of the user. The raw signals may contain noise or irrelevant information that may require further processing to extract meaningful insights.

In an implementation of the present disclosure, after obtaining the set of target data, the processing unit [106] performs on the set of target data at least one of a data pre-processing technique and a data segmentation technique to generate a pre-processed set of target data and a segmented set of target data. The data pre-processing technique may refer to the techniques used for pre-processing the electrical signals of raw data in the set of target data in order to enhance the quality of the set of target data, for example removal of noise from the set of target data to generate the pre-processed set of target data. The data segmentation technique may refer to the techniques used for segmenting large chunks of raw data signals into smaller, manageable units, for example, by dividing the set of target data into time-based segments such as for every 2 seconds to generate the segmented set of target data corresponding to every 2 seconds.

The data pre-processing technique as disclosed herein shall not be construed in any manner to limit the scope of the present disclosure and the data pre-processing technique may also include any other similar technique that a person skilled in the art while referring to the present disclosure may consider obvious for pre-processing the set of target data.

In an exemplary implementation of the present disclosure, for pre-processing the set of movement data, the data pre-processing technique may include a gravity-bias removal technique and a spurious motion removal technique. The gravity-bias removal technique may include removal of gravity-bias by applying a low-pass Butterworth filter that may attenuate signals below a gravity-bias-predefined-frequency such as attenuating the set of movement data having frequency lower than 0.5Hz. The spurious motion removal technique may include applying a high-pass filter that may attenuate signals above a spurious-motion-predefined-frequency such as the set of movement data having frequency above 20Hz. The set of movement data may be pre-processed by removing gravity-bias and removing spurious motion. In another exemplary implementation of the present disclosure, after the set of movement data may be pre-processed by removing the gravity-bias and spurious motion, a set of pre-processed movement data may be generated based on the set of movement data that may fall between the gravity-bias-predefined-frequency and the spurious-motion-predefined-frequency, for example, the set of pre-processed movement data may comprise the set of movement data having frequency between 0.5 Hz to 20 Hz.

In another implementation of the present disclosure, for pre-processing the set of muscle-activity data, the data pre-processing technique may include a band-pass filter technique, a notch filter technique, a full-wave rectification technique, a trend-analysis technique, a moving average envelope technique, a physiological threshold matching technique, a motion artifact removal technique and an interpolation drop technique. The band-pass filter technique may include applying a band-pass filter on the set of muscle-activity data that allows a predefined range of frequency and filters out data that falls outside the predefined range, for example the set of muscle-activity data having frequency between 20Hz and 450Hz may be kept while attenuating the set of muscle-activity data below 20Hz and above 450Hz. The notch filter technique may include applying a notch filter that attenuates the set of muscle-activity data having a predefined notch frequency, for example, attenuating the set of muscle-activity data having frequency of 50Hz and/or 60Hz. The full-wave rectification technique may include converting the set of muscle-activity data to positive range of values by removing all negative range of values from the set of muscle-activity data.

Further, the trend-analysis technique may include identifying a pattern of the set of movement data to understand a movement associated with a particular muscle/ a particular group of muscles. The moving average envelope analysis technique may include enhancing the set of muscle-activity data such as based on amplitude variations of moving averages. The physiological threshold matching technique may include matching the set of muscle-activity data with a physiological threshold criterion, wherein the physiological threshold criterion may be utilised to determine if a particular muscle/group of muscles are currently engaged by the user while performing a physical activity. The motion artifact removal technique may include removal of motion artifacts such as using the set of movement data to negate motion artifacts present within the set of muscle-activity data. The interpolation drop technique may include estimating gaps in the set of muscle-activity data that may be caused due to a poor-quality data or a missing data. In another exemplary implementation of the present disclosure, after pre-processing the set of muscle-activity data based on the data pre-processing techniques, a set of pre-processed muscle-activity data may be generated.

In another implementation of the present disclosure, for pre-processing the set of heart-rate data, the data pre-processing techniques may further include the band-pass filter technique and the motion-artifact removal technique. The set of heart-rate data may be pre-processed by applying a band-pass filter and cancelling motion-artifacts based on the band-pass filter technique and the motion-artifact removal technique. In an example, by detecting a movement of the PPG sensor [114] away from the skin of the user, and a variation in the heart-rate data caused due to such movement, a part of the set of heart-rate data may be cancelled as a motion artifact. In an exemplary implementation of the present disclosure, after pre-processing the set of heart-rate data based on the data pre-processing technique, a set of pre-processed heart-rate data may be generated.

In another implementation of the present disclosure, for pre-processing the set of temperature data, the data pre-processing technique may include the moving average envelope technique and a drift removal technique. The moving average envelope technique is utilised to enhance the set of temperature data by determining a moving average value associated with the set of temperature data based on the amplitude variations in the sensed set of temperature data over a predefined window of time for example, amplitude variation for every 10 seconds. The drift removal technique may include subtracting a baseline drift from the set of temperature data, for example, by removing a gradual temperature shift in the set of temperature data over a particular period of time. In an exemplary implementation of the present disclosure, after pre-processing the set of temperature data based on the data pre-processing techniques, a set of pre-processed temperature data may be generated.

In one example, the set of movement data may comprise an accelerometer data and a gyroscope data that may be used for identification of the position and orientation of the user, for example, the position and orientation of the user may indicate that a user is moving a hand in an upward direction. In one example, the IMU [110] may obtain the set of movement data at a predefined frequency ranging from 50 - 200 Hz.

Further, in another example, the EMG sensor [112] may sense the set of muscle-activity data such as analogue signals associated with activity of the user. Then, for pre-processing the set of muscle-activity data, the data pre-processing technique may further comprise an amplification technique, a filtering technique and a digitisation technique that may be performed on the analogue signals. For filtering technique, a powerline noise may be removed by applying the notch filter technique. The amplification techniques may be utilised for amplifying the set of muscle-activity data, and may include polymerase chain reaction, loop-mediated isothermal amplification, rolling circle amplification, and other such like amplification techniques. The digitisation techniques may be utilised for digitising the analogue signals to digital signals, and may include quantizing, sampling, encoding, and other such like digitisation techniques.

In one exemplary implementation of the present disclosure, the data segmentation technique may include a sliding window segmentation technique, task-agnostic segmentation technique, and flagging motion events technique. The sliding window segmentation technique may include dividing the set of target data into a set of sequential smaller segment such as in a predefined time window having a predefined overlap, for example, the set of target data may be divided into the set of sequential smaller segments having a time window of 1 second with an overlap of 50 percentage. It may be appreciated by the person skilled in the art that the above-mentioned segmentation techniques are exemplary in nature and any other such like segmentation techniques that may be obvious to a person skilled in the art may be utilised to extract the set of target features from the set of target data in accordance with the present disclosure.

Continuing further, after obtaining the set of target data, the processing unit [106] is configured to extract a set of target features from the set of target data. The set of target features comprises at least two from among a set of movement features, a set of muscle-activity features, a set of heart-rate features and a set of temperature features. The set of target features may be extracted from the set of target data by applying certain predefined operations and functions. In an implementation of the present disclosure, the set of movement features may be extracted from the set of movement data, the set of muscle-activity features may be extracted from the set of muscle-activity data, the set of heart-rate features may be extracted from the set of heart-rate data, and the set of temperature features may be extracted from the set of temperature data. In another implementation of the present disclosure, the set of movement features may be extracted from the set of pre-processed movement data, the set of muscle-activity features may be extracted from the set of pre-processed muscle-activity data, the set of heart-rate features may be extracted from the set of pre-processed heart-rate data, and the set of temperature features may be extracted from the set of pre-processed temperature data.

The set of movement features may refer to features that define the movement of the wearable device [100] and may be indicative of the a movement of the user, for example, the set of pre-processed movement data may indicate an upward movement of the wearable device [100] worn by a particular user on their wrist, and after extracting the upward movement indication, the set of movement features may be extracted and may indicate that such particular user has lifted their hand in the upwards direction at particular time. Similarly, the set of muscle-activity features may define muscle-activity like contraction of a set of muscles, for example, the set of muscle-activity data may indicate a periodic movement of a long head of a particular muscle such as a brachii muscle, associated with said particular user, the set of muscle-activity features would be indicative of muscle activation levels, muscle fatigue, etc. Further, the set of heart-rate features may define a rate at which the heart of the user is pumping blood at particular time, such set of heart-rate features indicates the blood circulation of the user at the particular time. Further, said set of heart-rate features may be utilised to determine the state of the user at the particular time i.e., the user state. For example, a high resting heart-rate may be indicative of stress and underlying conditions. Furthermore, the set of temperature features may define the temperature of skin that can indicate the body heat, hydration levels, etc. For example, an exemplary set of movement features may indicate that a user lifted their hands at a particular time period, an exemplary set of muscle-activity features may indicate that bicep muscles were contracted in the said time period, an exemplary set of heart-rate data may indicate that the heart-rate of the user was heightened during said time period, and an exemplary set of temperature data may indicate that the skin temperature of the user was also increased during said time period.

In an exemplary implementation of the present disclosure, the set of movement features may be extracted based on performing at least one of a mean acceleration operation, a variance operation, a signal magnitude area (SMA) operation, a tilt angle variance operation, a tremor frequency spectrum operation, an amplitude operation and a spectral entropy operation on the set of movement data. The mean acceleration operation may be utilised to determine a mean value(s) associated with the set of movement data, wherein the mean value(s) indicate a rate of change of a velocity parameter associated with the wearable device [100]. The variance operation may be utilised to determine an amount of dispersion in the set of movement data, wherein the amount of dispersion indicates how the set of movement data is spread out from an average value(s). The SMA operation may be utilised to quantify an overall strength of each of the set of movement data. The tilt angle variance operation may be utilised to determine a change in angles at which the wearable device may be tilted. The tremor frequency spectrum operation may be utilised to determine a tremor frequency associated with the set of movement data, wherein the tremor frequency may indicate a frequency of tremor associated with the wearable device [100]. The amplitude operation may be utilised to determine a measurement of intensity or strength associated with a movement of the wearable device [100]. The spectral entropy operation may be utilised to determine a spectral power distribution and a predictability of the set of movement data, wherein the power distribution and the predictability indicate a complexity of the set of movement data. The mean acceleration operation and variance operation is utilised to analyse a movement of the wearable device [100] in a particular direction such as the mean acceleration operation and the variance operation may be used to analyse a steady movement of the wearable device [100] in an upwards direction. The tilt angle variation operation may help in analysing the degree of change or fluctuation in the tilt angle of the wearable device [100], and the SMA operation may help in analysing the intensity of the movement, for example, the tilt angle variation operation may indicate a 360-degree rotational movement of the wearable device [100] indicating a rotation of a hand of a particular user (if the wearable device [100] is wore on the wrist of the user), and the SMA operation may indicate a speed of such movement of the wearable device [100]. The tremor frequency spectrum operation may help in analysing the tremors (or frequent shaking of the wearable device [100]) that are experienced by the user, the analysis of such tremors may be further used to identify issues associated with the user such as Parkinson's disease, etc. The spectral entropy operation may identify irregularity of the frequency of the movement, for example, if the set of movement data indicates that the wrist of the user is being rotated, a repetition pattern associated with such rotation movement may be determined by performing the spectral entropy operation on the set of movement data associated with said rotation movement.

In another implementation of the present disclosure, the set of muscle-activity features are extracted based on performing at least one of a mean absolute value (MAV) operation, a zero-crossing rate operation, a median frequency operation, a root mean square (RMS) operation, a muscle co-activation index operation, a spectral centroid operation and/or a spectral entropy operation on the set of muscle-activity data. The MAV operation may be utilised to determine a mean absolute value associated with the set of muscle-activity data, wherein the MAV may be indicative of a strength of the set of muscle-activity data. The zero-crossing rate operation may be utilised to determine a zero-crossing rate associated with the set of muscle-activity data, wherein the zero-crossing rate may be indicative of a frequency of the set of movement data in crossing a zero-amplitude point. The zero-crossing rate may help in analysis of muscle activation/ deactivation pattern and assessment of fatigue of the muscles. The median frequency operation may be utilised to determine a median frequency associated with the set of muscle-activity data, wherein changes in the median frequency by a predefined threshold value indicates a muscle state such a decrease in the median frequency by the predefined threshold value indicates that a fatigue is experienced by the user in a particular muscle i.e., a muscle utilised by the user at the particular time. The RMS operation may be utilised to determine a root mean square (RMS) value associated with the set of muscle-activity data, wherein the RMS value is indicative of a magnitude of force applied to activate a particular muscle. The muscle co-activation index operation may be utilised to determine a muscle co-activation index associated with the set of muscle-activity data, wherein the muscle co-activation index may be indicative of a set of muscles that may contract/relax during a particular movement. The spectral centroid operation may be utilised to determine a concentration of frequency associated with the set of muscle-activity data, wherein the concentration of frequency may be used to track changes in muscle activity over time, or compare activity across different muscles or individuals. The spectral entropy operation may be utilised to determine a spectral power distribution and a predictability of the set of muscle-activity data, wherein the power distribution and the predictability indicate a particular feature of the set of muscle-activity data, and such feature may be used to analyse muscle fatigue, pain and spinal stabilization while muscles are being engaged.

In another implementation of the present disclosure, the set of heart-rate features may be extracted based on performing at least one of an instantaneous heart rate operation, a standard deviation of a normal-to-normal intervals (SDNN) operation, a root mean square of successive differences (RMSSD) operation, a low frequency-high frequency ratio operation, a pulse-amplitude variability operation and a micro-vasculature variability operation on the set of heart-rate data. The instantaneous heart rate operation may be utilised to determine a heart-rate of the user at a particular time associated with the user, wherein the instantaneous heart-rate may indicate a beat-to-beat heart rate in real-time and a variation in the beat-to-beat heart rate may indicate a heart-rate variability. The SDNN operation may be utilised to determine a SDNN value associated with the set of heart-rate data, wherein the SDNN value may be indicative of a variability of heart-rate within a particular period of time. The RMSSD operation may be utilised to determine an RMSSD value associated with the set of heart-rate data, wherein the RMSSD value may be indicative of the variability of heart-rate within a particular time period. The SDNN operation and the RMSSD operation may be used for analysing the variability in heart rate and may indicate stress, sleep, exercises, and health conditions. The low frequency-high frequency ratio operation may be utilised to determine a low frequency-high frequency ratio associated with the set of heart-rate data, wherein the low frequency-high frequency ratio is indicative of cardiovascular health, autonomic functions, and responses to various stimuli. The micro-vasculature variability operation may be utilised to determine a micro-vasculature variability associated with the set of heart-rate data, wherein the micro-vasculature variability may help in detection of blood flow, vessel diameter changes, and other factors impacting blood volume in the smaller vessels, and also helps in identifying various conditions such as hypertension, diabetes, and peripheral artery disease. Further, the pulse amplitude variability operation may be utilised to determine a pulse amplitude variability associated with the set of heart-rate data, wherein the pulse amplitude variability may be indicative of a fluctuations or changes in the height/amplitude of the set of heart-rate data over a particular period of time.

In another implementation of the present disclosure, the set of temperature features may be extracted based on performing at least one of a temperature change over window operation on the set of temperature data. The temperature change over window operation may refer to a determination of a variation in temperature for a predefined time window, for example checking variation in temperature for every 20 seconds. The change in temperature helps analyse the energy expenditure and hydration levels of the user. For example, a high temperature may indicate high energy expenditure and lack of hydration levels, such high temperature may be caused due to exuberant physical activity that may performed by the user at a particular time such as running activity and a low-hydration levels because of high body sweat experienced by the user at the particular time due to said exuberant physical activity.

Once the set of features are extracted, the processing unit [106] is configured to generate a fused set of features based on a combination of two or more target features from the set of target features. In an exemplary implementation of the present disclosure, the fused set of features may be generated based on integrating or combining two or more features from the set of movement features, the set of muscle-activity features, the set of heart-rate features and/or the set of temperature features.

In an implementation of the present disclosure, the fused set of features is generated by the processing unit [106] based on a target technique using a first artificial-intelligence (AI)-based subsystem [118]. The first AI-based subsystem [118] may be a trained model or a fine-tuned model which may be specifically trained/fine-tuned using one or more machine learning techniques to generate the fused set of features from the set of target features. In an exemplary embodiment of the present disclosure, the first AI-based subsystem [118] may be a lightweight support vector machine (SVM) model, a tiny convolutional neural network (CNN) model, a lightweight random forest model, a long short-term memory (LSTM) model, and other like model.

In an implementation of the present disclosure, the target technique may be one or more of a concatenation technique, a normalization technique, a principal component analysis (PCA) technique, a dimensionality reduction technique, and/or a trend-cluster analysis technique. The concatenation technique may refer to a technique which combines together the target set of features to generate the fused set of features. The normalization technique may refer to a technique which normalizes or rescales the set of target features within a specific range. The PCA technique and the dimensionality reduction technique may refer to the techniques that transforms high-dimensional data into a smaller set of uncorrelated variables. The trend cluster analysis technique involves identifying and analysing groups of data points that exhibit similar trends or patterns over time. The target technique as disclosed above shall not be construed in any manner to limit the scope of the present disclosure and the target technique may also include any other similar technique that may be considered obvious to the person skilled in the art for generating the fused set of features to implement the present disclosure.

In the exemplary use-case scenario for detecting a fatigue level of the user in real-time, the fused set of features are generated by concatenating the set of target features and in another exemplary use- case scenario, the fused set of features are generated by applying the PCA technique to reduce the dimensions of the set of target features to 3-5 dimensions.

In the exemplary use-case scenario for neuromuscular disorder detection, the set of movement features, the set of muscle-activity features, the set of heart-rate features and the set of temperature features are fused to generate a fused set of features. For example, the fused set of features may be generated from the set of target features based on the that set of target techniques. The set of target techniques may include standard deviation operation that may be utilised to transform each feature based on the difference between a mean value and a standard deviation value. Then, the set of target techniques may also include a concatenating technique, wherein the concatenating technique may include concatenating of the set of target features to form a single, combined feature matrix. The set of target techniques may also include a PCA technique, wherein the PCA technique may be utilised to apply to reduce dimensions of the set of target features to that capture the most variance in the data that may be outputted as the fused set of features.

In an implementation of the present disclosure, the first AI-based subsystem [118] is trained based on at least one of a historical fused set of features generated from a historical set of target features. The historical set of features may refer to the set of target features that may be extracted from the set of target data in the past and stored in a repository. For example, an exemplary set of historical features may indicate that a user lifted their hands at a particular past time period, a bicep muscles of the user were contracted at the particular past time period, the heart-rate was heightened at the particular time period, and the skin temperature of the user was increased at the particular time period. The historical fused set of features may refer to the fused set of features that may be generated in the past and stored in the repository. For example, an exemplary fused set of features may indicate that a user lifted their hands to perform an exercise involving bicep muscles in the past. The historical fused set of features and the historical set of target features may be prestored in a repository which may be used to train the first AI-based subsystem [118]. Further, the historical set of target features comprises at least two from among a historical set of movement features, a historical set of muscle-activity features, a historical set of heart-rate features and a historical set of temperature features. For example, the historical set of movement features may indicate that a lifting movement is performed by a user in a particular time period in the past, the historical set of muscle-activity features may indicate that bicep muscles were contracted in the past time period, the historical set of heart-rate data may indicate that the heart-rate of the user was heightened in the past time period, and the historical set of temperature data may indicate that the skin temperature of the user was also increased in the past time period. In an exemplary implementation of the present disclosure, the historical set of movement features, the historical set of muscle-activity features, the historical set of heart-rate features and the historical set of temperature features may be used for training the first AI-based subsystem [118]. For example, based on the historical set of target features, the first AI-based subsystem [118] may analyse that for a particular time in the past, a particular historical set of target features were fused to generate the historical set of fused features, and using such analysis on a similar set of target features, it may generate a similar fused set of features.

Once the fused set of features are generated, the processing unit [106] is configured to classify the fused set of features into one or more categories. The one or more categories include a fatigue level category, a user activity category, and a user health category. The fatigue level category indicates levels of fatigue of the user, the user activity category indicates the activity performed by the user, and the user health category indicates the factors affecting health of the user. For example, if the fused set of features indicate that the user has been working out for a particular time period, and some muscles of the user has started to flinch at the particular time period, it may indicate the fatigue level category. In another example, if the fused set of features indicate that a user is repeatedly lifting their hands in a particular manner at a particular period of time, the bicep muscles are being contracted during the particular period of time, and the heart-rate and the temperature of the user is rising at the particular period of time, then it may indicate that a user is performing a bicep workout as the user activity category. For example, based on a pattern of contraction and relaxation of long head and short head of the bicep muscles, it may also classify the fused set of features as a hammer curl activity or a bicep curl activity to specifically distinguish each type of workout. In another example, if the user has been sleeping, but the heart-rate and temperature of the user is increased, it may indicate situations of stress, illness, etc. For example, if the fused set of features indicate a resting body, occurrence of muscle tremors, etc, it may also indicate certain diseases of the user.

In one implementation of the present disclosure, the fused set of features may be classified into one or more categories using a second artificial-intelligence (AI) based subsystem [120]. The second AI-based subsystem [120] may be a trained model or a fine-tuned model that may be specifically trained/fine-tuned using one or more machine learning techniques to classify the fused set of features into the one or more categories. In an implementation of the present disclosure, the second AI-based subsystem [120] may be an embedded in/integrated with the first AI-based subsystem [118]. In another implementation of the present disclosure, the second AI-based subsystem [120] may be part of the first AI-based subsystem [118] or may be integrated with the first AI-based subsystem [118]. In an exemplary embodiment of the present disclosure, the second AI-based subsystem [120] may be a lightweight support vector machine (SVM) model, a tiny convolutional neural network (CNN) model, a lightweight random forest model, a long short-term memory (LSTM) model, and other like model.

In another implementation of the present disclosure, the one or more historical categories may be classified based on the historical fused set of features. The one or more historical categories may be the one or more categories formed based on the historical fused set of features. For example, for a historical fused set of features indicating that the user was repeatedly lifting their hands in a particular manner at a particular time in the past, the bicep muscles were being contracted at the particular time the past, and the heart-rate and the temperature of the user was also rising at the particular time in the past, then for such historical fused set of features, the one or more historical categories were classified as the historical user activity category at such particular time in the past. The one or more historical categories may comprise a historical fatigue level category, a historical user activity category and a historical user health category. The historical fatigue level category may indicate the levels of fatigue of the user in the past, the historical user activity category may indicate the activities performed by the user in the past, and the historical user health category may indicate the factors affecting the health of the user in past. For example, a longer period of workout in the past indicated a high fatigue category as the historical fatigue level category, a repeated muscle contraction may indicate a muscle building workout activity as the historical user activity category in another particular time in past, and an occurrence of tremor experienced by the user in the past indicated a Parkinson's disease as the historical user health category at another particular time period in the past.

In an implementation of the present disclosure, the second AI-based subsystem [120] is trained based on one or more historical categories. The one or more historical categories may refer to the one or more categories formed in the past. In an exemplary implementation of the present disclosure, based on the historical set of fused features, the second AI-based subsystem [120] may analyse that in the past when the user was repeatedly lifting their hands and bicep muscles were contracted, the user activity category of bicep workout was classified, based on which, the second AI-based subsystem [120] may be trained to classify a similar fused set of features into a similar category. The one or more historical categories may also be prestored in a repository which may be used to train the second AI-based subsystem [120]. The second AI-based subsystem [120] may be trained based on the historical fatigue level category, the historical user activity category and the historical user health category.

On classification of the fused set of features into the one or more categories, the processing unit [106] is configured to determine the user state based on the one or more categories. For example, if the one or more categories indicate the fatigue level of the user such as high fatigue, the user state may be determined as a state of tiredness such as due to a heavy workout, or a muscle fatigue etc. In an implementation of the present disclosure, the fatigue level of the user is determined based on the fatigue level category, the movement of the user is determined based the user activity category, the activity of one or more muscles of the user is determined based on the user activity category, and the health state of the user is determined based on the user health category.

In the exemplary use-case scenario for detecting a fatigue level of the user in real-time, the fused set of features may be categorised into the one or more categories using the second AI-based subsystem [120], wherein the one or more categories may comprise a no-fatigue level category, an onset fatigue level category and/or a high-fatigue category. Also, in one implementation, based on the category, a feedback medium such as a haptic buzz or an on-screen indicator may be provided. In another implementation, the feedback medium may be an on-screen indicator such as in case of the onset fatigue level category and the high fatigue level category.

In the exemplary use-case scenario for neuromuscular disorder detection, the fused set of features may be categorised into the one or more categories using the second AI-based subsystem [120], wherein the one or more categories may comprise a no neuropathy category, a possible neuropathy category and/or a flag for clinical review category. Thereafter, in one implementation, based on the category, a feedback medium such as a weekly summary notification may be provided. In another implementation, the feedback medium may be a prompt to schedule a clinical consultation such as in case of the possible neuropathy or the flag for clinical review category.

In another use-case scenario, the set of movement data, the set of muscle-activity data, the set of heart-rate data and the set of temperature data may be downloaded in form of full-resolution logs and may be offloaded to a server. The server may reside in a wearable device [100], the wearable apparatus [200], a user equipment and/or a network entity. Thereafter, the set of movement data, the set of muscle-activity data, the set of heart-rate data and the set of temperature data may be pre-processed. Thereafter, the set of muscle-activity features are extracted such as by using spectral centroid, spectral entropy, etc., and the set of heart-rate features are extracted such as by using low frequency-high frequency ratio. Also, using the data from multiple sensors, coherence between the set of movement data and the set of muscle-activity data is extracted. Thereafter, the one or more categories are accumulated in a repository over each session.

Further, in another use-case scenario, for forecasting fatigue levels, the set of target data may be collected and pre-processed using the set of data pre-processing techniques and/or the set of data segmentation techniques. Thereafter, the set of target data may be used to extract the set of target features such as the heart rate variability, Optical Vital-sign node (OVN), and total activity counts cumulative load and recovery indices, etc. Further, for classifying or predicting a risk of fatigue level category for a subsequent period of time, a second AI-based subsystem [120] is utilised. Thereafter, based on the classified category, if risk of fatigue level category is greater than a predefined threshold a feedback medium may indicate an alert or a recommendation suggesting a reduced load or taking a rest day.

In an exemplary implementation of the present disclosure, for classifying or predicting the risk of fatigue level category, the second AI-based subsystem [120] may determine a fatigue risk score based on the set of muscle-activity features. For example, the second AI-based subsystem [120] may be configured for utilising the RMS operation to extract the set of muscle-activity features, in an event an increase in an RMS value associated with an activation of a particular muscle for performing a same activity determined based on a predefined threshold RMS value by the second AI-based subsystem [120], then such increase may indicate that an additional force is being applied for activation of the particular muscle to compensate for a muscle fatigue. Further, such increase in the RMS value may be utilised by the second AI-based subsystem [120] to determine a higher fatigue risk score. Similarly in another example, the second AI-based subsystem [120] may be configured for utilising the MAV operation and the median frequency operation for extracting the muscle-activity features, wherein a shift in a mean absolute value and a decline in a median frequency may indicate a localized muscle fatigue and the higher fatigue risk score. Such higher fatigue risk score may be then further used for classifying/predicting the risk of fatigue level category. Furthermore, the second AI-based subsystem [120] may be configured for by combine/fuse the set of target features such as the set of movement features, the set of muscle-activity features, the set of heart-rate features and the set of temperature features to predict/classify different scenarios to accurately indicate the fatigue level category.

For example, in case the fatigue risk score above a predefined fatigue risk score value (i.e., the higher fatigue risk score) which indicates a high load on the user, the set of heart-rate features indicate towards a low heart rate variability, and the set of muscle-activity features indicate towards a declining median frequency, then the fatigue level category may be classified as the high fatigue level category. Further, such high fatigue level category may indicate that the user is experiencing both central and peripheral fatigue and may recommend the user to take a rest day. Further, in another example, in case the higher fatigue risk score indicates the high load on the user, the set of heart-rate features indicate towards a stable heart rate variability, and the set of muscle-activity features indicate towards a declining median frequency, then the fatigue level category may be classified as the high fatigue level category and may recommend the user to switch to a different activity. Furthermore, in another example, in case the fatigue risk score indicates a low load on the user, the set of heart-rate features indicate towards a low heart rate variability, then the fatigue level category may be classified as no-fatigue level category indicating that the user is not physically tired, but may be mentally stressed or not fully recovered.

It may be appreciated by the person skilled in the art that the above implementation(s) for classifying or predicting the risk of fatigue level category and subsequently generating recommendations for the user based on the determined fatigue risk score is exemplary in nature and the same is provided solely for illustrative purposes and should not be interpreted as limiting the scope of potential methods, approaches, or systems for assessing fatigue risk or providing related recommendations in any manner.

In another exemplary use-case scenario of the present disclosure, for detecting sleep apnea, the data collection module [108] may collect the set of target data and heuristically extract the set of target features such as based on data pre-processing technique and/or the data segmentation techniques. The first AI-based subsystem [118] may be trained based on a data prestored related to patients having sleep apnea. Then, based on the second AI-based subsystem [120], the sleep apnea may be classified as the user health category indicating signs of sleep apnea. The data prestored related to patients may be generated based on signals obtained from throughout the sleep of users that may be patients of sleep apnea and then using medical grade techniques, the first AI-based subsystem [118] and second AI-based subsystem [120] may be trained to measure/ detect sleep apnea.

Similarly, in another implementation of the use-case scenario of detecting stress, the first AI-based subsystem [118] may be trained based on prestored data related to stress, that may be determined from EEG data from brain signals. Then, based on the second AI-based subsystem [120], stress levels and strain levels on brain may be classified as the user health category. The prestored data may be generated based on a sample collected from the users throughout a day and then using medical grade techniques, the first AI-based subsystem [118] and second AI-based subsystem [120] may be trained to measure stress.

Additionally, in another implementation of the use-case scenario of neuromuscular disorder detection, the first AI-based subsystem [118] may be trained based on a prestored data related to diagnosed movement disorders that may be tracked through muscular distortion tracking during sleep. Then, based on the second AI-based subsystem [120], muscular distortions may be classified as the user health category. The prestored data may be generated based on a sample collected from the users throughout a day and then using medical grade techniques, the first AI-based subsystem [118] and the second AI-based subsystem [120] may be trained to measure stress.

In another implementation of the use-case scenario of neuromuscular disorder detection, the present disclosure may be implemented for clinical assessment support. The set of target data may be offloaded as high-fidelity data and secure upload of raw streams to cloud server upon clinic arrival. Further, the set of target data may be pre-processed for artifact rejection and quality control. For pre-processing, the spike/spurious motion are removed for example, the data in which the change in acceleration is more than 4g are kept. Further, the set of muscle-activity data and the set of heart-rate data are pre-processed based on physiological thresholds and interpolate drops. Then the pre-processed data is aligned and segmented according to protocol-driven tasks. Thereafter, the set of muscle-activity features may be extracted such as by using spectral centroid, bandwidth and tremor coherence. Further, the set of heart-rate features may be extracted such as by using low frequency-high frequency ratio and baroreflex sensitivity, the set of heart-rate features are extracted. Also, using the data from multiple sensors, cross correlation between the set of movement data and the set of muscle-activity data is extracted. Thereafter, using a regression model as the first AI-based subsystem [118] and/or the second AI-based subsystem [120], clinical scales for diseases such as unified Parkinson's disease may be classified as the user health category based on clustering to differentiate tremor-dominant vs bradykinesia profiles. Further, the visual timelines of symptomatic episodes may be displayed as the feedback medium and automated report with quantitative scores as the feedback medium.

Further, in another implementation of the use-case scenario of neuromuscular disorder detection, the present disclosure may be implemented for longitudinal monitoring and treatment efficacy. The set of target data may be summarized such as daily and weekly. Thereafter, the tremor burden, muscle activation asymmetry and trends in heart rate variability may be extracted from the set of target features. Thereafter, exponential smoothening of severity scores and a long short-term memory model may be used for classifying or forecasting flare-up risk over the next few days. Thereafter, based on the classification, alerts and therapy tuning provided in the form of feedback medium. if risk is greater than a predefined threshold a feedback medium may indicate a push alert(s) or a recommendation suggesting adjusting of medication timing or intensity.

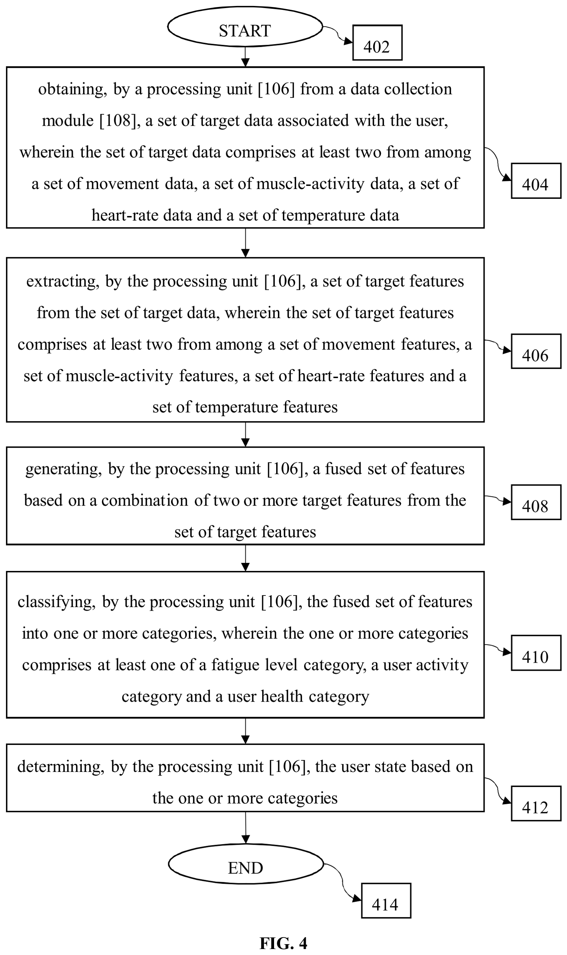

**FIG. 4** illustrates a method [400] for detecting muscle activity of a user, in accordance with exemplary implementations of the present disclosure. In an implementation, the method [400] is performed a wearable device [100]. The method [400] starts at **step [402].** In an implementation, the method [400] starts when a user gives a command for detection of muscle activity of the user. In another implementation, the method [400] starts based on preset/ dynamic commands received at the wearable device [100]. In another implementation, the method [400] automatically starts in an event of detecting an activity performed by the user of the wearable device [100].

The user may wear the wearable device [100] on any part of their body for detecting the user state through the wearable device [100]. As used herein the term "user state" may refer to a physiological state of the user that indicates the health and fitness of the user. Further, as used herein the term "physiological state" may refer to the condition of the body's physical functions encompassing various processes incurred by user's body and their impact on overall well-being of the user, for example working out for longer period of time may result in sustained exertion by the user indicating fatigue as the user state, similarly, higher resting heart-rate may indicate stress as the user state.

In an implementation of the present disclosure, the user state may indicate at least one of a fatigue level for the user, a movement of the user, an activity of one or more muscles of the user and a health state of the user. The fatigue level for the user may refer to a level of tiredness experienced by the user or in any body part of the user, for example, high tiredness, muscle twitching, etc. The movement of the user indicates the activity performed by the user, for example, a workout movement, a running movement, etc. The activity of the one or more muscles of the user may refer to an activity performed by a particular group of muscles forming a part of the body, for example, a bicep workout, a legs workout, etc. The health state of the user may refer to a condition of the body part of the user that indicates the healthiness of a particular body part, for example, frequent muscle twitching may indicate bad health state of the user.

At **step [404],** the method [400] comprises obtaining, by a processing unit [106] from a data collection module [108], a set of target data associated with the user, wherein the set of target data comprises at least two from among a set of movement data, a set of muscle-activity data, a set of heart-rate data and a set of temperature data. In one exemplary implementation of the present disclosure, the data collection module [108] may comprise a shared clock or a synchroniser that helps match the time at which the data is sensed by one or more sensors of the set of sensors [122]. In another exemplary implementation of the present disclosure, the set of movement data, the set of muscle-activity data, the set of heart-rate data and the set of temperature data are raw signals received from the set of sensors [122]. For example, the set of movement data may be collected from the IMU [110], the set of muscle-activity data may be collected from the EMG sensor [112], the set of heart-rate data may be collected from the PPG sensor [114], the set of temperature data may be collected from the temperature sensor [116]. The raw signal may refer to an electrical signal associated with the user that may be sensed by the set of sensors [122] during the detection of the user state of the user. The raw signals may contain noise or irrelevant information that may require further processing to extract meaningful insights.

In an implementation of the present disclosure, after obtaining the set of target data, the method [400] may further comprise, performing, by the processing unit [106] on the set of target data at least one of a data pre-processing technique and a data segmentation technique to generate a pre-processed set of target data and a segmented set of target data. The data pre-processing technique may refer to the techniques used for pre-processing the electrical signals of raw data in the set of target data in order to enhance the quality of the set of target data, for example removal of noise from the set of target data to generate the pre-processed set of target data. The data segmentation technique may refer to the techniques used for segmenting large chunks of raw data signals into smaller, manageable units, for example, by dividing the set of target data into time-based segments such as for every 2 seconds to generate the segmented set of target data corresponding to every 2 seconds.

The data pre-processing technique as disclosed herein shall not be construed in any manner to limit the scope of the present disclosure and the data pre-processing technique may also include any other similar technique that a person skilled in the art while referring to the present disclosure may consider obvious for pre-processing the set of target data.

In an exemplary implementation of the present disclosure, for pre-processing the set of movement data, the data pre-processing technique may include a gravity-bias removal technique and a spurious motion removal technique. The gravity-bias removal technique may include removal of gravity-bias by applying a low-pass Butterworth filter that may attenuate signals below a gravity-bias-predefined-frequency such as attenuating the set of movement data having frequency lower than 0.5Hz. The spurious motion removal technique may include applying a high-pass filter that may attenuate signals above a spurious-motion-predefined-frequency such as the set of movement data having frequency above 20Hz. The set of movement data may be pre-processed by removing gravity-bias and removing spurious motion. In another exemplary implementation of the present disclosure, after the set of movement data may be pre-processed by removing the gravity-bias and spurious motion, a set of pre-processed movement data may be generated based on the set of movement data that may fall between the gravity-bias-predefined-frequency and the spurious-motion-predefined-frequency, for example, the set of pre-processed movement data may comprise the set of movement data having frequency between 0.5 Hz to 20 Hz.

In another implementation of the present disclosure, for pre-processing the set of muscle-activity data, the data pre-processing technique may include a band-pass filter technique, a notch filter technique, a full-wave rectification technique, a trend-analysis technique, a moving average envelope technique, a physiological threshold matching technique, a motion artifact removal technique and an interpolation drop technique. The band-pass filter technique may include applying a band-pass filter on the set of muscle-activity data that allows a predefined range of frequency and filters out data that falls outside the predefined range, for example the set of muscle-activity data having frequency between 20Hz and 450Hz may be kept while attenuating the set of muscle-activity data below 20Hz and above 450Hz. The notch filter technique may include applying a notch filter that attenuates the set of muscle-activity data having a predefined notch frequency, for example, attenuating the set of muscle-activity data having frequency of 50Hz and/or 60Hz. The full-wave rectification technique may include converting the set of muscle-activity data to positive range of values by removing all negative range of values from the set of muscle-activity data. The trend-analysis technique may include identifying a pattern of the set of movement data to understand a movement associated with a particular muscle/ a particular group of muscles. The moving average envelope analysis technique may include enhancing the set of muscle-activity data such as based on amplitude variations of moving averages. The physiological threshold matching technique may include matching the set of muscle-activity data with a physiological threshold criterion, wherein the physiological threshold criterion may be utilised to determine if a particular muscle/group of muscles are currently engaged by the user while performing a physical activity. The motion artifact removal technique may include removal of motion artifacts such as using the set of movement data to negate motion artifacts present within the set of muscle-activity data. The interpolation drop technique may include estimating gaps in the set of muscle-activity data that may be caused due to a poor-quality data or a missing data. In another exemplary implementation of the present disclosure, after pre-processing the set of muscle-activity data based on the data pre-processing techniques, a set of pre-processed muscle-activity data may be generated.

In another implementation of the present disclosure, for pre-processing the set of heart-rate data, the data pre-processing techniques may further include the band-pass filter technique and the motion-artifact removal technique. The set of heart-rate data may be pre-processed by applying a band-pass filter and cancelling motion-artifacts based on the band-pass filter technique and the motion-artifact removal technique. In an example, by detecting a movement of the PPG sensor [114] away from the skin of the user, and a variation in the heart-rate data caused due to such movement, a part of the set of heart-rate data may be cancelled as a motion artifact. In an exemplary implementation of the present disclosure, after pre-processing the set of heart-rate data based on the data pre-processing technique, a set of pre-processed heart-rate data may be generated.

In another implementation of the present disclosure, for pre-processing the set of temperature data, the data pre-processing technique may include the moving average envelope technique and a drift removal technique. The moving average envelope technique is utilised to enhance the set of temperature data by determining a moving average value associated with the set of temperature data based on the amplitude variations in the sensed set of temperature data over a predefined window of time for example, amplitude variation for every 10 seconds. The drift removal technique may include subtracting a baseline drift from the set of temperature data, for example, by removing a gradual temperature shift in the set of temperature data over a particular period of time. In an exemplary implementation of the present disclosure, after pre-processing the set of temperature data based on the data pre-processing techniques, a set of pre-processed temperature data may be generated.

In one example, the set of movement data may comprise an accelerometer data and a gyroscope data that may be used for identification of the position and orientation of the user for example, the position and orientation of the user may indicate that a user is moving a hand in an upward direction. In one example, the IMU [110] may obtain the set of movement data at a predefined frequency ranging from 50 - 200 Hz.

Further, in another example, the EMG sensor [112] may sense the set of muscle-activity data such as analogue signals associated with activity of the user. Then, for pre-processing the set of muscle-activity data, the data pre-processing technique may further comprise an amplification technique, a filtering technique and a digitisation technique that may be performed on the analogue signals. For filtering technique, a powerline noise may be removed by applying the notch filter technique. The amplification techniques may be utilised for amplifying the set of muscle-activity data, and may include polymerase chain reaction, loop-mediated isothermal amplification, rolling circle amplification, and other such like amplification techniques. The digitisation techniques may be utilised for digitising the analogue signals to digital signals, and may include quantizing, sampling, encoding, and other such like digitisation techniques.

In one exemplary implementation of the present disclosure, the data segmentation technique may include a sliding window segmentation technique, task-agnostic segmentation technique, and flagging motion events technique. The sliding window segmentation technique may include dividing the set of target data into a set of sequential smaller segment such as in a predefined time window having a predefined overlap, for example, the set of target data may be divided into the set of sequential smaller segments having a time window of 1 second with an overlap of 50 percentage. It may be appreciated by the person skilled in the art that the above-mentioned segmentation techniques are exemplary in nature and any other such like segmentation techniques that may be obvious to a person skilled in the art may be utilised to extract the set of target features from the set of target data in accordance with the present disclosure.

Continuing further, after obtaining the set of target data, at **step [406],** the method [400] comprises, extracting, by the processing unit [106], a set of target features from the set of target data, wherein the set of target features comprises at least two from among a set of movement features, a set of muscle-activity features, a set of heart-rate features and a set of temperature features. The set of target features may be extracted from the set of target data by applying certain predefined operations and functions. In an implementation of the present disclosure, the set of movement features may be extracted from the set of movement data, the set of muscle-activity features may be extracted from the set of muscle-activity data, the set of heart-rate features may be extracted from the set of heart-rate data, and the set of temperature features may be extracted from the set of temperature data. In another implementation of the present disclosure, the set of movement features may be extracted from the set of pre-processed movement data, the set of muscle-activity features may be extracted from the set of pre-processed muscle-activity data, the set of heart-rate features may be extracted from the set of pre-processed heart-rate data, and the set of temperature features may be extracted from the set of pre-processed temperature data.

The set of movement features may refer to features that define the movement of the wearable device [100] and may be indicative of the a movement of the user, for example, the set of pre-processed movement data may indicate an upward movement of the wearable device [100] worn by a particular user on their wrist, and after extracting the upward movement indication, the set of movement features may be extracted and may indicate that such particular user has lifted their hand in the upwards direction at particular time. Similarly, the set of muscle-activity features may define muscle-activity like contraction of a set of muscles, for example, the set of muscle-activity data may indicate a periodic movement of a long head of a particular muscle such as a brachii muscle, associated with said particular user, the set of muscle-activity features would be indicative of muscle activation levels, muscle fatigue, etc. Further, the set of heart-rate features may define a rate at which the heart of the user is pumping blood at particular time, such set of heart-rate features indicates the blood circulation of the user at the particular time. Further, said set of heart-rate features may be utilised to determine the state of the user at the particular time i.e., the user state. For example, a high resting heart-rate may be indicative of stress and underlying conditions. Furthermore, the set of temperature features may define the temperature of skin that can indicate the body heat, hydration levels, etc. For example, an exemplary set of movement features may indicate that a user lifted their hands at a particular time period, an exemplary set of muscle-activity features may indicate that bicep muscles were contracted in the said time period, an exemplary set of heart-rate data may indicate that the heart-rate of the user was heightened during said time period, and an exemplary set of temperature data may indicate that the skin temperature of the user was also increased during said time period.

In an exemplary implementation of the present disclosure, the set of movement features may be extracted based on performing at least one of a mean acceleration operation, a variance operation, a signal magnitude area (SMA) operation, a tilt angle variance operation, a tremor frequency spectrum operation, an amplitude operation and a spectral entropy operation on the set of movement data. The mean acceleration operation may be utilised to determine a mean value(s) associated with the set of movement data, wherein the mean value(s) indicate a rate of change of a velocity parameter associated with the wearable device [100]. The variance operation may be utilised to determine an amount of dispersion in the set of movement data, wherein the amount of dispersion indicates how the set of movement data is spread out from an average value(s). The SMA operation may be utilised to quantify an overall strength of each of the set of movement data. The tilt angle variance operation may be utilised to determine a change in angles at which the wearable device may be tilted. The tremor frequency spectrum operation may be utilised to determine a tremor frequency associated with the set of movement data, wherein the tremor frequency may indicate a frequency of tremor associated with the wearable device [100]. The amplitude operation may be utilised to determine a measurement of intensity or strength associated with a movement of the wearable device [100]. The spectral entropy operation may be utilised to determine a spectral power distribution and a predictability of the set of movement data, wherein the power distribution and the predictability indicate a complexity of the set of movement data. The mean acceleration operation and variance operation is utilised to analyse a movement of the wearable device [100] in a particular direction such as the mean acceleration operation and the variance operation may be used to analyse a steady movement of the wearable device [100] in an upwards direction. The tilt angle variation operation may help in analysing the degree of change or fluctuation in the tilt angle of the wearable device [100], and the SMA operation may help in analysing the intensity of the movement, for example, the tilt angle variation operation may indicate a 360-degree rotational movement of the wearable device [100] indicating a rotation of a hand of a particular user (if the wearable device [100] is wore on the wrist of the user), and the SMA operation may indicate a speed of such movement of the wearable device [100]. The tremor frequency spectrum operation may help in analysing the tremors (or frequent shaking of the wearable device [100]) that are experienced by the user, the analysis of such tremors may be further used to identify issues associated with the user such as Parkinson's disease, etc. The spectral entropy operation may identify irregularity of the frequency of the movement, for example, if the set of movement data indicates that the wrist of the user is being rotated, a repetition pattern associated with such rotation movement may be determined by performing the spectral entropy operation on the set of movement data associated with said rotation movement.

In another implementation of the present disclosure, the set of muscle-activity features are extracted based on performing at least one of a mean absolute value (MAV) operation, a zero-crossing rate operation, a median frequency operation, a muscle co-activation index operation, a spectral centroid operation and/or a spectral entropy operation on the set of muscle-activity data. The MAV operation may be utilised to determine a mean absolute value associated with the set of muscle-activity data, wherein the MAV may be indicative of a strength of the set of muscle-activity data. The zero-crossing rate operation may be utilised to determine a zero-crossing rate associated with the set of muscle-activity data, wherein the zero-crossing rate may be indicative of a frequency of the set of movement data in crossing a zero-amplitude point. The zero-crossing rate may help in analysis of muscle activation/ deactivation pattern and assessment of fatigue of the muscles. The median frequency operation may be utilised to determine a median frequency associated with the set of muscle-activity data, wherein changes in the median frequency by a predefined threshold value indicates a muscle state such a decrease in the median frequency by the predefined threshold value indicates that a fatigue is experienced by the user in a particular muscle i.e., a muscle utilised by the user at the particular time. The muscle co-activation index operation may be utilised to determine a muscle co-activation index associated with the set of muscle-activity data, wherein the muscle co-activation index may be indicative of a set of muscles that may contract/relax during a particular movement. The spectral centroid operation may be utilised to determine a concentration of frequency associated with the set of muscle-activity data, wherein the concentration of frequency may be used to track changes in muscle activity over time, or compare activity across different muscles or individuals. The spectral entropy operation may be utilised to determine a spectral power distribution and a predictability of the set of muscle-activity data, wherein the power distribution and the predictability indicate a particular feature of the set of muscle-activity data, and such feature may be used to analyse muscle fatigue, pain and spinal stabilization while muscles are being engaged.

In another implementation of the present disclosure, the set of heart-rate features may be extracted based on performing at least one of an instantaneous heart rate operation, a standard deviation of a normal-to-normal intervals (SDNN) operation, a root mean square of successive differences (RMSSD) operation, a low frequency-high frequency ratio operation, a pulse-amplitude variability operation and a micro-vasculature variability operation on the set of heart-rate data. The instantaneous heart rate operation may be utilised to determine a heart-rate of the user at a particular time associated with the user, wherein the instantaneous heart-rate may indicate a beat-to-beat heart rate in real-time and a variation in the beat-to-beat heart rate may indicate a heart-rate variability. The SDNN operation may be utilised to determine a SDNN value associated with the set of heart-rate data, wherein the SDNN value may be indicative of a variability of heart-rate within a particular period of time. The RMSSD operation may be utilised to determine an RMSSD value associated with the set of heart-rate data, wherein the RMSSD value may be indicative of the variability of heart-rate within a particular time period. The SDNN operation and the RMSSD operation may be used for analysing the variability in heart rate and may indicate stress, sleep, exercises, and health conditions. The low frequency-high frequency ratio operation may be utilised to determine a low frequency-high frequency ratio associated with the set of heart-rate data, wherein the low frequency-high frequency ratio is indicative of cardiovascular health, autonomic functions, and responses to various stimuli. The micro-vasculature variability operation may be utilised to determine a micro-vasculature variability associated with the set of heart-rate data, wherein the micro-vasculature variability may help in detection of blood flow, vessel diameter changes, and other factors impacting blood volume in the smaller vessels, and also helps in identifying various conditions such as hypertension, diabetes, and peripheral artery disease. Further, the pulse amplitude variability operation may be utilised to determine a pulse amplitude variability associated with the set of heart-rate data, wherein the pulse amplitude variability may be indicative of a fluctuations or changes in the height/amplitude of the set of heart-rate data over a particular period of time.

In another implementation of the present disclosure, the set of temperature features may be extracted based on performing at least one of a temperature change over window operation on the set of temperature data. The temperature change over window operation may refer to a determination of a variation in temperature for a predefined time window, for example checking variation in temperature for every 20 seconds. The change in temperature helps analyse the energy expenditure and hydration levels of the user. For example, a high temperature may indicate high energy expenditure and lack of hydration levels, such high temperature may be caused due to exuberant physical activity that may performed by the user at a particular time such as running activity and a low-hydration levels because of high body sweat experienced by the user at the particular time due to said exuberant physical activity.

Once the set of features are extracted, at **step [408],** the method [400] further comprises generating, by the processing unit [106], a fused set of features based on a combination of two or more target features from the set of target features. In an exemplary implementation of the present disclosure, the fused set of features may be generated based on integrating or combining two or more features from the set of movement features, the set of muscle-activity features, the set of heart-rate features and/or the set of temperature features.

In an implementation of the present disclosure, the fused set of features is generated by the processing unit [106] based on a target technique using a first artificial-intelligence (AI)-based subsystem [118]. The first AI-based subsystem [118] may be a trained model or a fine-tuned model which may be specifically trained/fine-tuned using one or more machine learning techniques to generate the fused set of features from the set of target features. In an exemplary embodiment of the present disclosure, the first AI-based subsystem [118] may be a lightweight support vector machine (SVM) model, a tiny convolutional neural network (CNN) model, a lightweight random forest model, a long short-term memory (LSTM) model, and other like model.

In an implementation of the present disclosure, the target technique may be one or more of a concatenation technique, a normalization technique, a principal component analysis (PCA) technique, a dimensionality reduction technique, and/or a trend-cluster analysis technique. The concatenation technique may refer to a technique which combines together the target set of features to generate the fused set of features. The normalization technique may refer to a technique which normalizes or rescales the set of target features within a specific range. The PCA technique and the dimensionality reduction technique may refer to the techniques that transforms high-dimensional data into a smaller set of uncorrelated variables. The trend cluster analysis technique involves identifying and analysing groups of data points that exhibit similar trends or patterns over time. The target technique as disclosed above shall not be construed in any manner to limit the scope of the present disclosure and the target technique may also include any other similar technique that may be considered obvious to the person skilled in the art for generating the fused set of features to implement the present disclosure.

In the exemplary use-case scenario for detecting a fatigue level of the user in real-time, the fused set of features are generated by concatenating the set of target features and in another exemplary use- case scenario, the fused set of features are generated by applying the PCA technique to reduce the dimensions of the set of target features to 3-5 dimensions.

In the exemplary use-case scenario for neuromuscular disorder detection, the set of movement features, the set of muscle-activity features, the set of heart-rate features and the set of temperature features are fused to generate a fused set of features. For example, the fused set of features may be generated from the set of target features based on the that set of target techniques. The set of target techniques may include standard deviation operation that may be utilised to transform each feature based on the difference between a mean value and a standard deviation value. Then, the set of target techniques may also include a concatenating technique, wherein the concatenating technique may include concatenating of the set of target features to form a single, combined feature matrix. The set of target techniques may also include a PCA technique, wherein the PCA technique may be utilised to apply to reduce dimensions of the set of target features to that capture the most variance in the data that may be outputted as the fused set of features.

In an implementation of the present disclosure, the first AI-based subsystem [118] is trained based on at least one of a historical fused set of features generated from a historical set of target features. The historical set of features may refer to the set of target features that may be extracted from the set of target data in the past and stored in a repository. For example, an exemplary set of historical features may indicate that a user lifted their hands at a particular past time period, a bicep muscles of the user were contracted at the particular past time period, the heart-rate was heightened at the particular time period, and the skin temperature of the user was increased at the particular time period. The historical fused set of features may refer to the fused set of features that may be generated in the past and stored in the repository. For example, an exemplary fused set of features may indicate that a user lifted their hands to perform an exercise involving bicep muscles in the past. The historical fused set of features and the historical set of target features may be prestored in a repository which may be used to train the first AI-based subsystem [118] . Further, the historical set of target features comprises at least two from among a historical set of movement features, a historical set of muscle-activity features, a historical set of heart-rate features and a historical set of temperature features. For example, the historical set of movement features may indicate that a lifting movement is performed by a user in a particular time period in the past, the historical set of muscle-activity features may indicate that bicep muscles were contracted in the past time period, the historical set of heart-rate data may indicate that the heart-rate of the user was heightened in the past time period, and the historical set of temperature data may indicate that the skin temperature of the user was also increased in the past time period. In an exemplary implementation of the present disclosure, the historical set of movement features, the historical set of muscle-activity features, the historical set of heart-rate features and the historical set of temperature features may be used for training the first AI-based subsystem [118] . For example, based on the historical set of target features, the first AI-based subsystem [118] may analyse that for a particular time in the past, a particular historical set of target features were fused to generate the historical set of fused features, and using such analysis on a similar set of target features, it may generate a similar fused set of features.

Once the fused set of features are generated, at **step [410],** the method [400] comprises classifying, by the processing unit [106], the fused set of features into one or more categories, wherein the one or more categories comprises at least one of a fatigue level category, a user activity category and a user health category. The fatigue level category indicates levels of fatigue of the user, the user activity category indicates the activity performed by the user, and the user health category indicates the factors affecting health of the user. For example, if the fused set of features indicate that the user has been working out for a particular time period, and some muscles of the user has started to flinch at the particular time period, it may indicate the fatigue level category. In another example, if the fused set of features indicate that a user is repeatedly lifting their hands in a particular manner at a particular period of time, the bicep muscles are being contracted during the particular period of time, and the heart-rate and the temperature of the user is rising at the particular period of time, then it may indicate that a user is performing a bicep workout as the user activity category. For example, based on a pattern of contraction and relaxation of long head and short head of the bicep muscles, it may also classify the fused set of features as a hammer curl activity or a bicep curl activity to specifically distinguish each type of workout. In another example, if the user has been sleeping, but the heart-rate and temperature of the user is increased, it may indicate situations of stress, illness, etc. For example, if the fused set of features indicate a resting body, occurrence of muscle tremors, etc, it may also indicate certain diseases of the user.

In one implementation of the present disclosure, the fused set of features may be classified into one or more categories using a second artificial-intelligence (AI) based subsystem [120]. The second AI-based subsystem [120] may be a trained model or a fine-tuned model that may be specifically trained/fine-tuned using one or more machine learning techniques to classify the fused set of features into the one or more categories. In an implementation of the present disclosure, the second AI-based subsystem [120] may be an embedded in/integrated with the first AI-based subsystem [118]. In another implementation of the present disclosure, the second AI-based subsystem [120] may be part of the first AI-based subsystem [118] or may be integrated with the first AI-based subsystem [118]. In an exemplary embodiment of the present disclosure, the second AI-based subsystem [120] may be a lightweight support vector machine (SVM) model, a tiny convolutional neural network (CNN) model, a lightweight random forest model, a long short-term memory (LSTM) model, and other like model.

In another implementation of the present disclosure, the one or more historical categories may be classified based on the historical fused set of features. The one or more historical categories may be the one or more categories formed based on the historical fused set of features. For example, for a historical fused set of features indicating that the user was repeatedly lifting their hands in a particular manner at a particular time in the past, the bicep muscles were being contracted at the particular time the past, and the heart-rate and the temperature of the user was also rising at the particular time in the past, then for such historical fused set of features, the one or more historical categories were classified as the historical user activity category at such particular time in the past. The one or more historical categories may comprise a historical fatigue level category, a historical user activity category and a historical user health category. The historical fatigue level category may indicate the levels of fatigue of the user in the past, the historical user activity category may indicate the activities performed by the user in the past, and the historical user health category may indicate the factors affecting the health of the user in past. For example, a longer period of workout in the past indicated a high fatigue category as the historical fatigue level category, a repeated muscle contraction may indicate a muscle building workout activity as the historical user activity category in another particular time in past, and an occurrence of tremor experienced by the user in the past indicated a Parkinson's disease as the historical user health category at another particular time period in the past.

In an implementation of the present disclosure, the second AI-based subsystem [120] is trained based on one or more historical categories. The one or more historical categories may refer to the one or more categories formed in the past. In an exemplary implementation of the present disclosure, based on the historical set of fused features, the second AI-based subsystem [120] may analyse that in the past when the user was repeatedly lifting their hands and bicep muscles were contracted, the user activity category of bicep workout was classified, based on which, the second AI-based subsystem [120] may be trained to classify a similar fused set of features into a similar category. The one or more historical categories may also be prestored in a repository which may be used to train the second AI-based subsystem [120]. The second AI-based subsystem [120] may be trained based on the historical fatigue level category, the historical user activity category and the historical user health category.

On classification of the fused set of features into the one or more categories, at **step [412],** the method [400] comprises, determining, by the processing unit [106], the user state based on the one or more categories. For example, if the one or more categories indicate the fatigue level of the user such as high fatigue, the user state may be determined as a state of tiredness such as due to a heavy workout, or a muscle fatigue etc. In an implementation of the present disclosure, the fatigue level of the user is determined based on the fatigue level category, the movement of the user is determined based the user activity category, the activity of one or more muscles of the user is determined based on the user activity category, and the health state of the user is determined based on the user health category.

Thereafter, the method [400] is terminated at **step [414].**

Yet another aspect of the present disclosure may relate to a non-transitory computer readable storage medium storing one or more instructions for detecting a user state of a user, the one or more instructions include executable code which, when executed by one or more units of a wearable device, causes a processing unit of the wearable device to obtain, from a data collection module, a set of target data associated with the user. The target data comprises at least two from among a set of movement data, a set of muscle-activity data, a set of heart-rate data and a set of temperature data. Further, the one or more instructions when executed causes the processing unit to extract a set of target features from the set of target data, wherein the set of target features comprises at least two from among a set of movement features, a set of muscle-activity features, a set of heart-rate features and a set of temperature features. Further, the one or more instructions when executed causes the processing unit to generate a fused set of features based on a combination of two or more target features from the set of target features. Further, the one or more instructions when executed causes the processing unit to classify the fused set of features into one or more categories. The one or more categories comprises at least one of a fatigue level category, a user activity category and a user health category. Further, the one or more instructions when executed causes the processing unit to determine the user state based on the one or more categories.

The wearable device [100] as disclosed herein for detecting a user state of a user and the method thereof (collectively referred to as "proposed solution") have several use cases for various purposes, which are explained in the foregoing description. The proposed solution as disclosed above provides enhanced muscle signal acquisition leading to accurate detection of the activity performed by the user even with minimal limb displacement such as a forearm movement or an upper arm placement. The wearable device [100] can also be adapted to be provided on smart clothing by embedding the wearable device [100] in a compression sleeve or a shirt. For example, the IMU [110] and the EMG sensor [112] of the wearable device [100] may be provided on the sleeve of the shirt, making the sleeve of the shirt the wearable device [100], and the shirt a wearable apparatus [200]. The wearable device [100] can also be adapted in form factor which may be utilised by the user to wrap the wearable device [100] around the ankle of the user so as to allow the user to track and monitor the activity in its leg muscles. Thus, the wearable device [100] enables tracking muscle activity in lower body muscle of the user and the movement patterns associated with such muscle activity. The wearable device [100] provided by the present solution can be adapted to be worn by the user over any muscle, which enables usability for different applications and purposes. Hence, the present disclosure provides a solution that can be wore dynamically by the user in different manner and does not restrict to a wearable device worn on the hand/wrist of the user.

Further, due to the integration of multiple sensors from the set of sensors [122] such as the IMU [110], the EMG sensor [112], the PPG sensor [114], and the temperature sensor [116] in accordance with the proposed solution, the detection and monitoring of muscle fatigue is improved, thus preventing overtraining and injuries to the user of the wearable device [100]. Further, the proposed solution improves real-time monitoring of muscle activity, motion, heart rate, and skin temperature of the user so as to enable in real-time a detection/identification of one or more health conditions such as sleep apnea episodes, fatigue level, neuromuscular disorder, stress, Parkinson's tremors, epileptic muscle spasms, bradykinesia, etc. that may facilitate early intervention in such cases. Further, the wearable device [100] enables real-time tracking of degradation in muscles, which may help in tracking conditions like amyotrophic lateral sclerosis (ALS) and muscular dystrophy.

Further, the wearable device [100] may detect the fall of the user in real-time by utilizing data from multiple sensors and may also indicate the effect of such a fall on the body of the user of the wearable device [100]. The wearable device [100] of the present disclosure may also be used for detection of tremors caused in the muscle. Further, the wearable device [100] of the present solution will enable monitoring of pain in the muscles of a user by utilizing data from multiple sensors that may be collected in real time and/or prestored sensor data associated with the user. Similarly, there may be various applications and use cases of the present invention, which may be understood by a person skilled in the art in light of the present disclosure. Hence, the present disclosure provides a solution that is capable of adapting to different use-cases, hence providing a single technical solution that is capable of performing multiple tasks including but not limited to muscle degradation detection, health conditions detection, activity detections, etc.

Hence, as it is evident from above, the present invention provides a technically advanced solution. The present invention combines the data signals from multiple sensors such as IMU, EMG sensor, PPG sensor and temperature sensor, that enables superior tracking of health, fatigue, workouts and activities performed by the user of the wearable device. The present invention enables monitoring of changes in muscle activation over time, which enables detection of the levels of fatigue of the user. The present invention provides a precise analysis of the muscle groups used during a physical activity performed by the user such as workouts that enables detection of even slight differentiation in the type of activity performed such as a type workout that is performed by the user. The present invention provides immediate exercise form analysis and counts repetition of the exercise, thereby enabling provision of real-time feedback. The present invention provides a solution for a wearable device that can be worn on different muscle groups based on the exercise needs. The present solution provides dynamic capability by not limiting the wearing of the wearable device over the wrist and allowing the wearable device to be worn on/placed in vicinity of different body parts of the user. Further, the present solution is capable of providing insights about different states of the user including but not limited to fatigue monitoring, post-workout fatigue monitoring, muscle degradation detection, health conditions detection for various diseases and disorders, accurate user activity detection, stress monitoring, clinical assessment support. Further, the present disclosure also provides a capability of providing real-time feedback such as by providing alerts, insights and recommendation to the user.

In the above detailed description, reference is made to the accompanying figure that forms a part thereof, and illustrates the best mode presently contemplated for carrying out the invention. However, such description should not be considered as any limitation of scope of the present invention. The structure of the elements thus conceived in the present description is susceptible of numerous modifications and variations/ embodiments, all the details may furthermore be replaced with elements having technical equivalence.

While considerable emphasis has been placed herein on the disclosed embodiments, it will be appreciated that many embodiments can be made and that many changes can be made to the embodiments without departing from the principles of the present disclosure. These and other changes in the embodiments of the present disclosure will be apparent to those skilled in the art, whereby it is to be understood that the foregoing descriptive matter to be implemented is illustrative and non-limiting.

## Claims

1. A wearable device [100] for detecting a user state of a user, the wearable device [100] comprising:
a data collection module [108] configured to collect data from a set of sensors [122]; and
a data processing module [102] connected at least to the data collection module [108], the data processing module [102] comprising a memory unit [104] and a processing unit [106], wherein the processing unit [106] is configured to:
obtain, from a data collection module [108], a set of target data associated with the user, wherein the target data comprises at least two from among a set of movement data, a set of muscle-activity data, a set of heart-rate data and a set of temperature data;
extract a set of target features from the set of target data, wherein the set of target features comprises at least two from among a set of movement features, a set of muscle-activity features, a set of heart-rate features and a set of temperature features;
generate a fused set of features based on a combination of two or more target features from the set of target features;
classify the fused set of features into one or more categories, wherein the one or more categories comprises at least one of a fatigue level category, a user activity category and a user health category; and
determine the user state based on the one or more categories.

2. The wearable device [100] as claimed in claim 1, wherein the user state indicates at least one of a fatigue level for the user, a movement of the user, an activity of one or more muscles of the user and a health state of the user, and wherein the fatigue level of the user is determined based on the fatigue level category, the movement of the user is determined based the user activity category, the activity of one or more muscles of the user is determined based on the user activity category, and the health state of the user is determined based on the user health category.

3. The wearable device [100] as claimed in claim 1 or 2, wherein the set of movement features is extracted from the set of movement data, the set of muscle-activity features is extracted from the set of muscle-activity data, the set of heart-rate features is extracted from the set of heart-rate data, and the set of temperature features is extracted from the set of temperature data.

4. The wearable device [100] as claimed in any of claims 1 to 3, wherein:
- the fused set of features is generated by the processing unit [106] based on a target technique using a first artificial-intelligence (AI)-based subsystem [118]; and
- the first AI-based subsystem [118] is trained based on at least one of a historical fused set of features generated from a historical set of target features, and wherein the historical set of target features comprises at least two from among a historical set of movement features, a historical set of muscle-activity features, a historical set of heart-rate features and a historical set of temperature features.

5. The wearable device [100] as claimed in any of claims 1 to 4, wherein:
- the one or more categories are generated by the processing unit [106] using a second artificial-intelligence (AI)-based subsystem [120]; and
- the second AI-based subsystem [120] is trained based on one or more historical categories, and wherein:
the one or more historical categories are classified based on a historical fused set of features, and
the one or more historical categories comprises at least one of a historical fatigue level category, a historical user activity category and a historical user health category.

6. The wearable device [100] as claimed in any of claims 1 to 5, wherein the data collection module [108] comprises the set of sensors [122] selected from among an inertial measurement unit (IMU) [110], an electromyography (EMG) sensor [112], a photoplethysmography (PPG) sensor [114], and a temperature sensor [116].

7. The wearable device [100] as claimed in any of claims 1 to 6, wherein prior to extracting the set of target features from the set of target data, the processing unit [106] is configured to perform at least one of a data pre-processing technique and a data segmentation technique on the set of target data.

8. A method for detecting a user state of a user, the method comprising:
obtaining, by a processing unit [106] from a data collection module [108], a set of target data associated with the user, wherein the set of target data comprises at least two from among a set of movement data, a set of muscle-activity data, a set of heart-rate data and a set of temperature data;
extracting, by the processing unit [106], a set of target features from the set of target data, wherein the set of target features comprises at least two from among a set of movement features, a set of muscle-activity features, a set of heart-rate features and a set of temperature features;
generating, by the processing unit [106], a fused set of features based on a combination of two or more target features from the set of target features;
classifying, by the processing unit [106], the fused set of features into one or more categories, wherein the one or more categories comprises at least one of a fatigue level category, a user activity category and a user health category; and
determining, by the processing unit [106], the user state based on the one or more categories.

9. The method as claimed in claim 8, wherein the user state indicates at least one of a fatigue level for the user, a movement of the user, an activity of one or more muscles of the user and a health state of the user, and wherein the fatigue level of the user is determined based on the fatigue level category, the movement of the user is determined based the user activity category, the activity of one or more muscles of the user is determined based on the user activity category, and the health state of the user is determined based on the user health category.

10. The method as claimed in claim 8 or 9, wherein the set of movement features is extracted from the set of movement data, the set of muscle-activity features is extracted from the set of muscle-activity data, the set of heart-rate features is extracted from the set of heart-rate data, and the set of temperature features is extracted from the set of temperature data.

11. The method as claimed in any of claims 8 to 10, wherein:
- the fused set of features is generated by the processing unit [106] based on a target technique using a first artificial-intelligence (AI)-based subsystem [118]; and
- the first AI-based subsystem [118] is trained based on at least one of a historical fused set of features generated from a historical set of target features, and wherein the historical set of target features comprises at least two from among a historical set of movement features, a historical set of muscle-activity features, a historical set of heart-rate features and a historical set of temperature features.

12. The method as claimed in any of claims 8 to 11, wherein:
- the one or more categories are generated by the processing unit [106] using a second artificial-intelligence (AI) based subsystem [120]; and
- the second AI-based subsystem [120] is trained based on one or more historical categories, and wherein:
the one or more historical categories are classified based on a historical fused set of features, and
the one or more historical categories comprises at least one of a historical fatigue level category, a historical user activity category and a historical user health category.

13. The method as claimed in any of claims 8 to 12, wherein the data collection module [108] comprises one or more sensors selected from among an inertial measurement unit (IMU) [110], an electromyography (EMG) sensor [112], a photoplethysmography (PPG) sensor [114], and a temperature sensor [116].

14. The method as claimed in any of claims 8 to 13, wherein prior to extracting the set of target features from the set of target data, the processing unit [106] performs on the set of target data at least one of a data pre-processing technique and a data segmentation technique.
